# EUROPÄISCHE PATENTSCHRIFT

(11) **EP 2 263 653 B1**
(45) Veröffentlichungstag und Bekanntmachung des Hinweises auf die Patenterteilung: **13.02.2013**
(21) Anmeldenummer: 10178676.2
(22) Anmeldetag: 23.12.2005
(51) Int. Cl.: B01F 9/00, A61K 9/16, A61K 9/127, A61Q 19/00, A61K 9/50, A61K 47/48, A61K 9/51, A61K 8/14

(54) **Verwendung einer dualen asymetrischen Zentrifuge (DAZ) zur Herstellung von lipidbasierten Nanopartikeln.**
Use of a dual asymetric centrifuge for the preparation of lipid based nano-particles.
Utilisation d'une centrifuge duale et asymétrique pour la préparation de nano-particules à bases lipides.

(30) Priorität: 23.12.2004 EP 04106939
(43) Veröffentlichungstag der Anmeldung: 22.12.2010
(62) Teilanmeldung aus: 05823526.8
(73) Patentinhaber: KTB Tumorforschungsgesellschaft mbH, 79106 Freiburg (DE)
(72) Erfinder: Massing, Ulrich, 79249 Merzhausen (DE)
(74) Vertreter: von Kreisler Selting Werner

(56) Entgegenhaltungen:
- DE-A1-102004 005 783
- DE-B- 1 151 718
- US-A- 6 099 160
- US-A- 6 126 097

## Beschreibung

Die Erfindung betrifft die Verwendung einer dualen asymmetrischen Zentrifuge (DAZ) und/oder eines Mischbehälters für eine DAZ zum Durchmischen von ein- oder mehrphasigen Gemischen, zum Zellaufschluss und zur Aufarbeitung von Geweben.

### Hintergrund der Erfindung

Lipidbasierte Nanopartikel wie z. B. Liposomen, Nano- und Mikroemulsionen, SLN (feste Lipidnanopartikel), Nanokapseln, Nanosphären und Lipoplexe sind wichtige Hilfsmittel für eine Vielzahl von technischen Prozessen und medizinischen Anwendungen.

Liposomen sind sphärische Gebilde, die aus Lipiden aufgebaut sind. In wässerigen Lösungen entstehen Liposomen durch Selbstaggregation dieser Lipide, wobei sich eine Lipiddoppelschicht bildet, die einen wässerigen Innenraum einschließt.

In Abhängigkeit von physikalischen Parametern wie mechanischen Einflüssen, Druck, Temperatur und der Ionenkonzentration sowie der vorhandenen Lipide und Zusatzstoffe, bilden sich unilamellare, oligolamellare oder multilamellare Liposomen. Die Liposomen können abhängig von ihren Bestandteilen eine positive oder negative Überschussladung tragen.

Liposomen können auch mit Substanzen beladen werden, die je nach Lipophilie oder Hydrophilie überwiegend in der Lipidschicht oder überwiegend im wässerigen Innenraum der Liposomen eingeschlossen sind. Derartige Liposomen werden in diagnostischen Nachweisverfahren, als therapeutische Mittel zum Transport von Wirkstoffen im Organismus oder als Wirkstoffdepot verwendet. Darüber hinaus können solche Liposomen auch In der biologischen und biomedizinischen Forschung und im Pflanzenschutz eingesetzt werden (z. B. zum Substanztransport in Zellen). Auch eine Anwendung in der Kosmetik ist möglich.

Die Eigenschaften der Liposomen wie beispielsweise ihre Stabilität oder Lagerfähigkeit werden wesentlich durch die in der Lipidschicht vorhandenen Substanzen bestimmt.

Zur Herstellung von Liposomen werden membranbildende Lipide, wie beispielsweise Phosphatidylcholin, Phosphatidylglycerin, Phosphatidylserin, Phosphatidylinositol und Phosphatldylethanolamin, Sphingomyelin, kationische Lipide wie beispielsweise DOTAP, DC-Chol, DOTMA u.a., membranbildende Amphiphile, wie beispielsweise Blockpolymere, Alkylester, -ether, -amide von Zuckern, Diolen und Polyolen, Aminen, Aminosäuren, Peptiden und Zuckern und Cholesterol sowie weitere Stoffe verwendet,

Zur Herstellung von möglichst einheitlichen leeren oder substanzbeladenen Liposomen sind verschiedene Verfahren bekannt. Diese Verfahren sind von Lasch et al. übersichtlich zusammengefasst worden (Lasch, J. et al., Preparation of Liposomes; in "Liposomes - a practical approach", Torchilin, V.P. und Weissig, V., Ed., 2. Aufl. (2003)).

Viele Herstellungsverfahren gehen von sog. "hand-shaken vesicles" aus, die durch einfache Rehydratisierung eines Lipidfilms und anschließendes Schütteln (meist in einem Kolben) hergestellt werden können. Diese Liposomen sind meist sehr uneinheitlich bezüglich ihrer Größe und ihrer Lamellarität. Mit den Verfahren der Extrusion, des Gefrier-Tau-Verfahrens oder der Ultrabeschallung können diese Liposomen in ihrer Größe vereinheitlicht (meist verkleinert) und ihrer Lamellarität verringert werden:
Extrusionsverfahren bedienen sich eines Extruders und ermöglichen in der Regel nur die Herstellung kleiner Mengen (Handextrusion) (Macdonald, R.E. et al., BBA 1061:297-303 (1991)). Die Herstellung großer Mengen ist mit hohem apparativen Aufwand (Pumpe, Membranen etc.) und einer aufwändigen Vorbereitung verbunden. Überdies können nur niedrigviskose Medien extrudiert werden, was zu einer niedrigen Einschlusseffizienz führt. Durch den offenen Umgang mit den Medien ist die Herstellung steriler Proben aufwändig und bei radioaktiven Materialien besteht die Gefahr der Kontamination der Umgebung. Lipidmischungen müssen außerdem vor der Extrusion und der Hydratation durch gemeinsames Lösen und anschließendes Evaporieren hergestellt werden.

Injektionsverfahren und Detergenzverfahren sind zwar großtechnisch möglich, aber die Lösungsmittelentfernung bzw. die Entfernung des Detergenz ist problematisch. Hohe Lipidkonzentrationen sind nicht einfach herstellbar, und durch den Überschuss an wässerigem Medium werden nur niedrige Einschlusseffizienzen bei hydrophilen Substanzen erreicht.

Auch durch Gefrier-Tau-Schritte können uneinheitliche, oft multilamellare Liposomen in ihrer Größe vereinheitlicht werden und ihre Lamellarität erniedrigt werden. Die Folge ist meist eine gesteigerte Einschlusseffizienz für wasserlösliche Substanzen.

Bei den bisher genannten Verfahren ist insbesondere die niedrige Einschlusseffizienz für hydrophile Substanzen ein systemimmanentes Problem. Dies begründet sich dadurch, dass diese Verfahren nur mit relativ geringen Lipidmengen durchführbar sind und folglich die gebildeten Liposomen nur einen geringen Anteil des wässerigen Gesamtvolumens einschließen können. Hydrophile, in der wässerigen Phase gelöste Substanzen werden folglich auch nur zu einem geringen Anteil eingeschlossen.

Die Einschlusseffizienz lässt sich z. B. durch eine Liposomen-Herstellung durch Hochdruckhomogenisation steigern, da hier erheblich höhere Lipidmengen eingesetzt werden können. Als Ergebnis erhält man bei sehr hohem Lipidanteil ein sogenanntes Liposomen-Gel, bei dem das wässerige Außenvolumen größenordnungsmäßig in etwa dem wässerigen Innenvolumen entspricht. Dementsprechend hoch ist dann auch der Anteil des eingeschlossenen hydrophilen Wirkstoffs. Ein weiterer Vorteil ist die Möglichkeit zur Herstellung von großen Mengen einer Formulierung, die mit Hilfe der Hochdruckhomogenisation einfach gelingt. Die Hochdruckhomogenisation hat zudem den Vorteil, insbesondere kleine Vesikel zu erzeugen, die u.a. im medizinischen Bereich von besonderem Interesse sind, z. B. für ein Tumortargeting unter Nutzung des sogenannten EPR-Effekts (enhanced permeability and retention). Dieser Effekt beruht auf der meist stark erhöhten vaskulären Permeabilität von Blutgefäßen in Tumoren. Durch die Undichtigkeit der Gefäße können kleine Partikel wie z. B. Liposomen (insbesondere wenn sie sehr klein sind) das Gefäßbett verlassen und sich im Tumor anreichern (Yuan, F. et al., Cancer Res. 54:3352-3356 (1994)).

Den wirtschaftlich besonders vorteilhaften Eigenschaften der Hochdruckhomogenisation (kleine Vesikel, hohe Einschlusseffizienz, große Probenmengen) steht eine Reihe von Nachteilen gegenüber:
Die Herstellung von sterilen Materialien, wie sie für die Anwendung an Mensch und Tier unerlässlich sind, ist problematisch. Eine sterile Herstellung ist zwar möglich, aber wegen der notwendigen Durchführung der Hochdruckhomogenisation unter Reinraumbedingungen oder der nachträglichen Autoklavierung des Materials aufwändig. Zusätzlich ergeben sich bei der Autoklavierung von wirkstoffhaltigen vesikulären Phospholipid Gelen (VPG) oft Probleme mit der Wirkstoff- und/oder der Lipidstabilität (Moog, R. et al., Int. J. Pharm. 206:43-53 (2000)).

Mit Hilfe der Hochdruckhomogenisation ist es insbesondere schwierig, kleine Probenmengen herzustellen, wie sie z. B. in medizinischen oder molekularbiologischen Laboren (wenn nur wenig Substanz zur Verfügung steht, bei Verwendung von radioaktiven Substanzen etc.) oder bei einem Screening von sehr vielen verschiedenen Lipidformulierungen (z. B. im Bereich der Präformulierung) benötigt werden.

Zudem wird die Probe durch den Homogenisierungsschritt stark belastet, was insbesondere bei teuren und empfindlichen Substanzen wie z. B. biologischen Materialien (DNA, siRNA, Antikörper, Peptide, Proteine) oder bei empfindlichen niedermolekularen Substanzen wie z. B. Antioxidantien, Lipiden mit bestimmten hochungesättigten Fettsäuren, Zytostatika (Alkylantien etc.) kritisch ist.

Die Ausrüstung für die Hochdruckhomogenisation ist teuer, groß und für viele (insbesondere medizinische/molekularbiologische) Labors nicht akzeptabel. Da bei jedem Wechsel der Zusammensetzung die Maschine gereinigt werden muss, ist der Probendurchsatz niedrig, ein Screening von verschiedenen Mischungen praktisch nicht möglich und nur schwer zu automatisieren (z. B. im Bereich Präformulierung).

Zudem ist überdurchschnittliches technologisches Know-how nötig, nicht zuletzt um die Gefahr einzuschränken, die bei Verwendung gefährlicher Substanzen (z. B. radioaktiver Substanzen oder Zytostatika) für die Umgebung besteht.

Die Probleme bei der Herstellung von Liposomen haben bereits dazu geführt, dass für kosmetische Zwecke Liposomen-Gele eingesetzt werden, welche nur durch Rehydratisierung einer Lipidmischung entstanden sind, wobei auf eine Vereinheitlichung der Vesikel völlig verzichtet wird (DE 10255285). Solche Formulierungen sind für pharmazeutische, biomedizinische und medizinische Zwecke nicht brauchbar und aufgrund der geringen Reproduzierbarkeit der Vesikelzusammensetzung für kosmetische Anwendungen zumindest bedenklich.

SLN sind Nanopartikel mit einer Größe von ca. 50 bis 1000 nm. Eine Übersicht über SLN wird in Pharmazeutische Technologie: Moderne Arzneiformen. R.H. Müller und G.E. Hildebrand, Wissenschaftliche Verlagsgesellschaft Stuttgart, 1998, gegeben. Das Matrixmaterial besteht - im Gegensatz zu Emulsionen - aus festen Lipiden. Hier werden überwiegend eingesetzt: physiologische Lipide (z. B. entsprechende Triglyceride) oder Lipide aus physiologischen Komponenten (z. B. Glyceride aus körpereigenen Fettsäuren). Es wird angenommen, das dadurch eine gute *in vivo* Verträglichkeit erreicht wird (Weyhers, H. et al., Proc. 1st world meeting APV/APGI, Budapest, 489-490 (1995)). Das Matrixmaterial ist aber nicht auf physiologische Lipide beschränkt, vorstellbar sind z. B. auch Wachse und nicht physiologische Triglyceride.

SLN werden bisher durch Hochdruckhomogenisation von in Wasser dispergierten Lipiden im geschmolzenen oder im festen Zustand hergestellt (Heiß- bzw. Kalthomogenisation; siehe Müller, R.H., Pharm. Ind. 49:423-427 (1997); EP 0 605 497; Schwarz, C. et al., J. Controlled Rel. 30:83-96 (1994); Müller, R.H. et al., Eur. J. Pharm. Biopharm. 41:62-69 (1995)). Diese Herstellungstechnik durch Hochdruckhomogenisation zeichnet sich dadurch aus, dass die SLN in ihrer Größe sehr homogen sind und zudem der Anteil an Mikropartikeln sehr gering ist. Der Aufwand für solch eine Hochdruckhornogenisation ist jedoch wie bei der Herstellung von Liposomen sehr hoch.

Ein weiterer Typ lipidbasierter Nanopartikel sind die Tröpfchen in Emulsionen, wobei hier Submikron-Emulsionen (SME) gemeint sind, also O/W-Emulsionen mit Tröpfchen/Partikelgrößen von unter 1 µm (Benita, S. et al., J. Pharm. Sci. 82:1069-1079 (1993)). Nicht davon abgegrenzt werden können sog. Nanoemulsionen mit Tröpfchengrößen von 50-1000 nm.

Solche Emulsionen werden seit langem zur parenteralen Ernährung eingesetzt (Lucks, J. S. et al., Krankenhauspharmazie 15:51-57 (1994)), können aber auch als Arzneistoffträger verwendet werden. Eine Übersicht zu SMEs findet sich in Pharmazeutische Technologie: Moderne Arzneiformen. R.H. Müller und G.E. Hildebrand, Wissenschaftliche Verlagsgesellschaft Stuttgart, 1998.

Unter den derzeit eingesetzten SME-Herstellungsverfahren ist die Hochdruckhomogenisation (durch Kolbenspalthomogenisatoren oder durch Microfluidizer-Technik) die führende Technik. Im Labor- und Technikumsmaßstab kommt auch die Ultraschallhomogenisation zum Einsatz, v.a. deswegen, weil die Hochdruckhomogenisation zu aufwendig ist.

WO 02/09677 beschreibt die Herstellung einer Dispersion, die eine O/W- oder W/O-Emulsion sowie einen in Öl und Wasser schwerlöslichen Wirkstoff (Amphotericin B) umfasst. Diese Dispersion kann eine Menge des Wirkstoffs enthalten, die höher ist als die Menge, die sich additiv aus seiner maximalen Löslichkeit in Wasser oder Öl ergibt. Die Homogenisierung erfolgt allerdings hochenergetisch, also durch Hochdruckhomogenisierung mit den damit verbundenen oben aufgeführten Nachteilen. Das Verfahren ist auch als sog. SolEmuls-Technologie beschrieben (Müller, R.H. et al., Int. J. Pharm. 269:293-302 (2004)). Hierbei werden mit Hilfe der hochenergetischen Hochdruckhomogenisation schwer lösliche Wirkstoffe wie Carbamazepin, Itraconazole oder Amphotericin B durch Co-Homogenisation in Emulsionen eingearbeitet, was zwar eine starke Steigerung insbesondere der Lösungsgeschwindigkeit zur Folge hat, aber auch die bereits benannten Nachteile einer Hochdruckhomogenisation besitzt.

Ein Verfahren zum Zertrümmern von Feststoffen mittels Kugelmühlen ist in DE-B-115171B offenbart. US 6,126,097 beschreibt eine Kugelmühle zum Zerkleinern von Feststoffen und US 6,099,160 beschreibt eine zentrifugenartige Mischvorrichtung.

### Kurzbeschreibung der Erfindung

Es wurde gefunden, dass sich eine duale asymmetrische Zentrifuge sehr gut zum Durchmischen von ein- oder mehrphasigen Gemischen, zum Zellaufschluss und zur Aufarbeitung von Geweben geeignet ist.

Die Erfindung umfasst somit
(1) Verwendung einer Mischvorrichtung für chemische und/oder biologische Substanzen mit
   einer ersten Antriebseinrichtung (12) zum Drehen eines Auslegers (16) um eine erste Drehachse (18),
   einem in einem Abstand zur ersten Drehachse (18) mit dem Ausleger (16) verbundenen Mischbehälter (40) zur Aufnahme der Substanzen und
   einer zweiten Antriebseinrichtung (30) zum Drehen des Mischbehälters (40) um eine zweite, durch den Mischbehälter (40) verlaufende Drehachse (28)
   wobei die Innenwände (46) des Mischbehälters (40) unterschiedliche Abstände zur zweiten Drehachse (28) aufweisen, und/oder
   wobei eine innerhalb des Aufnahmebehälters (24) angeordnete Halteeinrichtung (72, 80) zum lagegenauen Halten des Mischbehälters (40) in dem Aufnahmebehälter (24) dient, und/oder
   eines Mischbehälters für eine dualen asymmetrischen Zentrifuge (DAZ) mit einem ersten Aufnahmeraum (50) zur Aufnahme einer ersten Substanz und mindestens einem zweiten Aufnahmeraum (52) zur Aufnahme einer zweiten Substanz, wobei in einer Trennwand (70) zwischen den Aufnahmeräumen (50, 52) eine Öffnung (68) vorgesehen ist, um beim Auftreten von Zentrifugalkräften ein Überführen einer der Substanzen in den anderen Aufnahmeraum (50, 52) zu ermöglichen, zur Herstellung von lipidbasierten Nono-partikeln
   zum Durchmischen von ein- oder mehrphasigen biologishe substauzen Zellaufschluss und zur Aufarbeitung von Geweben in einer DAZ;
(2) eine bevorzugte Ausführungsform von (1), wobei
   (i) das Durchmischen bzw. der Zellaufschluss bzw. die Aufarbeitung von Gewebe bei wenigstens 200 rpm, bevorzugt bei wenigstens 1000 rpm und maximal 4000 rpm, besonders bevorzugt bei 2000 bis 3540 rpm, am bevorzugtesten bei 2500 bis 3540 rpm erfolgt; und/oder
   (ii) die g-Zahl wenigstens 1,2 g, bevorzugt wenigstens 80 g, besonders bevorzugt wenigstens 300 g, ganz besonders bevorzugt von 550 bis 1000 g oder von 620 bis 1500 g beträgt; und/oder
   (iii) das Rückdrehverhältnis von 1:6 bis 6:1 beträgt, bevorzugt kleiner als 5:1, besonders bevorzugt kleiner als 3:1 ist; und/oder
   (iv) die Zentrifugationszeit von 30 s bis 1 h, bevorzugt von 1 bis 30 min, besonders bevorzugt von 3 bis 20 min beträgt; und/oder
   (v) eine Mischhilfe, bevorzugt Glasperlen mit einem Durchmesser von 0,5 bis 6 mm, verwendet wird;
(3) ein Verfahren zur Zerkleinerung von Feststoffen, insbesondere zum Aufschluss von Zellen oder Gewebe, umfassend die Behandlung der Feststoffe in einer DAZ; und
(4) einen Kit zur Durchführung des Verfahrens gemäß (3), umfassend die Halteeinrichtung (72, 88), wie in (1) definiert.

### Kurzbeschreibung der Figuren

Die Erfindung wird anhand der nachfolgenden Figuren in der detaillierten Beschreibung der Erfindung näher erläutert.
- Fig. 1:: Schematischer Aufbau einer dualen asymmetrischen Zentrifuge in Aufsicht
- Fig. 2:: Schematischer Aufbau einer dualen asymmetrischen Zentrifuge in Seitenansicht
- Fig. 3:: Mischbehälter für Mischvorrichtung der Ausführungsform (10)
- Fig. 4:: Mischbehälter mit Öffnung und zwei Aufnahmeräumen gemäß Ausführungsform (11)

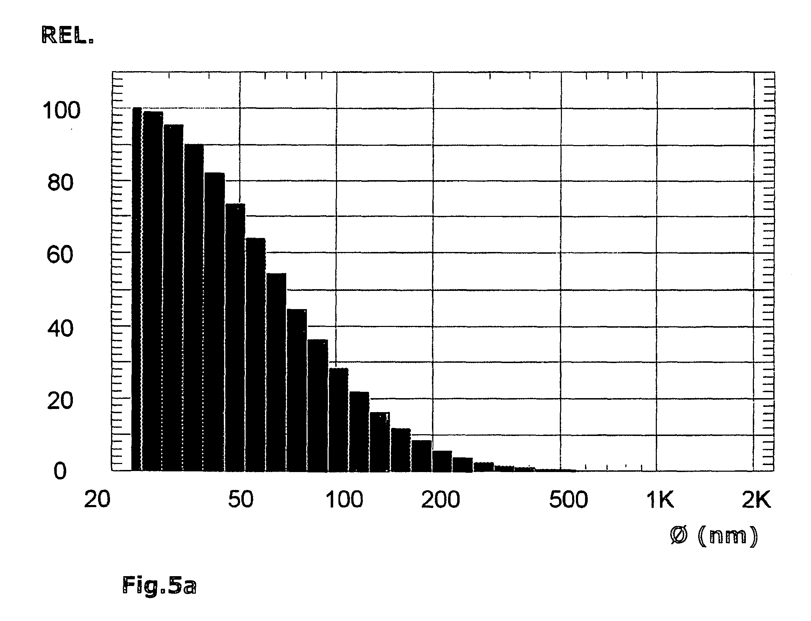

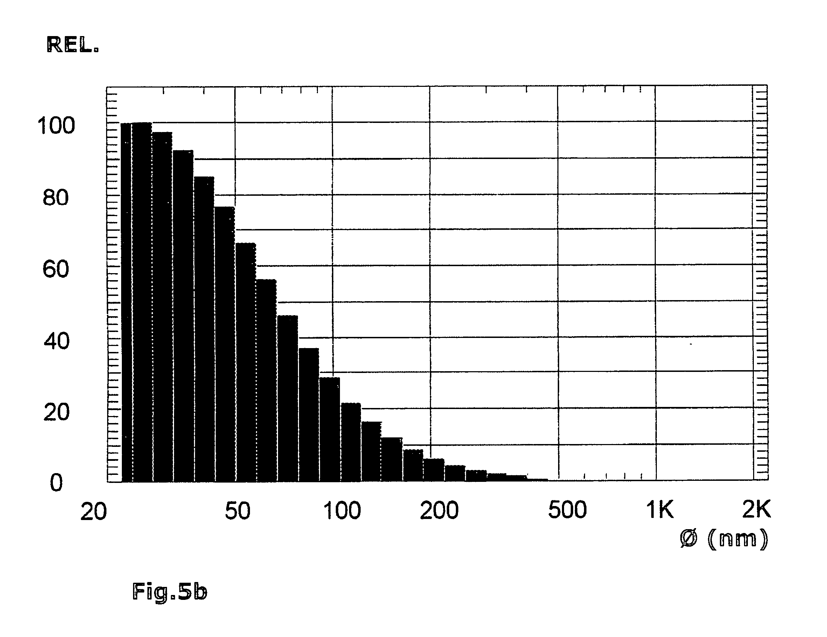

- Fig. 5:: Partikelgrößenverteilung von Liposomen (Bsp. 1, vial 2 und 3), bestimmt als relative Gauß-Verteilung bezogen auf die Teilchenzahl; Diam: Vesikeldurchmesser; Rel: relativer Anteil in %: a) Herstellung mit DAZ (vial 2) und b) Herstellung durch Hochdruckhomogenisation (vial 3)
- Fig. 6:: Einfluss verschiedener Parameter auf die Partikelgröße bei Speedmixen in einer Injektionsflasche (vgl. Bsp. 18): A) Variation der Lipidkonzentration; B) Unterschiedliche Speedmix-Zeiten und C) Glasperlen unterschiedlicher Größe als Dispergierhilfe
- Fig. 7:: Einfluss der Speedmixer-Geschwindigkeit auf die Partikelgröße
- Fig.8:: Mischvorrichtung der Ausführungsform (13) zum Speedmixen einer Injektionsflasche
- Fig. 9:: Schematische Schnittansicht der in Fig. 8 dargestellten Ausführungsform
- Fig. 10:: Mischbehälter zur Aufnahme von Kryo-Vials oder Eppendorfgefäßen, der bei Verwendung geeigneter Materialien wie Teflon, PE UHMW, Aluminium auch tieftemperaturgeeignet ist
- Fig. 11:: Schnittansicht entlang der Linie XI-XI in Fig. 10
- Fig. 12:: Schematische perspektivische Ansicht des in den Fig. 10 und 11 dargestellten Mischbehälters

### Detaillierte Beschreibung der Erfindung

Eine "duale asymmetrische Zentrifuge" (DAZ) ist eine Zentrifuge, bei der zusätzlich zu den Vorgängen einer klassischen Zentrifugation das Gefäß mit dem zu zentrifugierenden Material bevorzugt entgegen der Drehbewegung der Zentrifuge gedreht wird, z. B. mit einem Viertel bis Drittel der Drehzahl der Zentrifuge. Dies führt zu einer stetigen Vermischung des in die DAZ eingebrachten Materials. Die hohe Zentripetalkraft erlaubt es auch, viskose Massen zu mischen (EP 1293245). Bei diesem Verfahren entsteht insbesondere in viskosen Massen eine starke innere Reibung. Die DAZ wird üblicherweise zur Vermischung von Pasten mit Pasten, Pasten mit Pulvern, Pulvern mit Pulvern etc. verwendet. Typische Einsatzgebiete sind die Anmischung von Dichtstoffen und Beschichtungsmitteln (wie z. B. Silikonen, Polyurethanen und Acrylaten), Lacken, Druckfarben und Pigmenten sowie das Vermischen von Ein-, Zwei- oder Multikomponenten-Produkten in flüssiger oder pastöser Form.

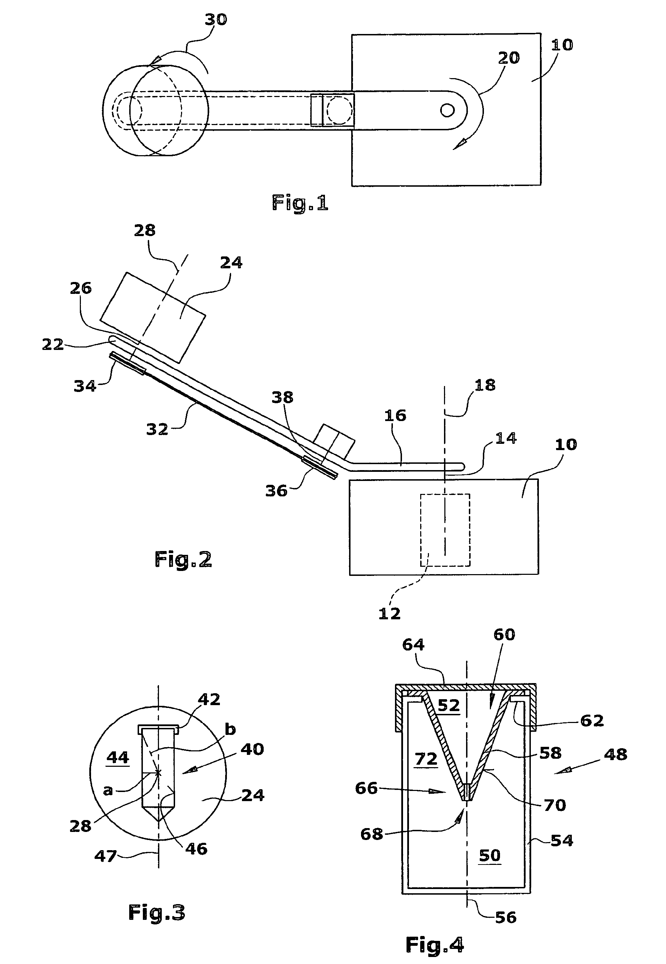

Die in der erfindungsgemäßen Verwendung (1) eingesetzte duale asymmetrische Zentrifuge wird im folgenden anhand der Fig. 1 und Fig. 2 näher beschrieben. Sie weist ein Gehäuse bzw. einen Basiskörper 10 auf. In dem Gehäuse 10 ist eine erste Antriebseinrichtung 12 in Form eines Elektromotors vorgesehen. Durch die erste Antriebseinrichtung 12 wird eine Antriebswelle 14 angetrieben. Mit der Antriebswelle 14 ist ein Ausleger 16 verbunden, der somit mit Hilfe der ersten Antriebseinrichtung um eine erste Drehachse 18 gedreht werden kann. Mit Hilfe der ersten Antriebseinrichtung 12 erfolgt somit ein Drehen des vorzugsweise abgewinkelt ausgebildeten Auslegers 16, beispielsweise in Richtung eines Pfeils 20. An einem von der Drehachse 18 beabstandeten Ende 22 des Auslegers 16 ist ein Behälter 24 vorgesehen. Der Behälter 24 ist über eine Welle 26 mit dem Ausleger 16 drehbar verbunden, so dass der Behälter 24 um eine zweite Drehachse 28, beispielsweise in Richtung eines Pfeils 30 drehbar ist. Zum Antrieb des Behälters 24 ist eine zweite Antriebseinrichtung 30 vorgesehen, die beispielsweise auf dem Ausleger 16 befestigt ist. Um die Einwirkung von Zentrifugalkräften auf die zweite Antriebseinrichtung 30 zu verringern, ist die zweite Antriebseinrichtung möglichst nahe an der ersten Drehachse 18 angeordnet. Der Antrieb erfolgt sodann über einen Keilriemen 32 sowie zwei entsprechende Keilriemenräder 34, 36, die mit der Welle 26 bzw. mit einer Motorwelle 38 verbunden sind.

Durch das gleichzeitige Drehen des Behälters 24 um die beiden Drehachsen 18 und 28 erfolgt ein Durchmischen von in dem Behälter 24 vorgesehenen Substanzen. Hierbei kann eine hohe Homogenität der Durchmischung erzielt werden. Vorzugsweise sind die beiden Drehachsen 18, 28 nicht nur in einem Abstand zueinander angeordnet, sondern weisen einen Winkel zueinander auf. Dieser kann durch Vorsehen eines abgewinkelten Auslegers 16 hervorgerufen werden. Ebenso ist es möglich, die Drehachse 28 in einem Winkel zum Ausleger anzuordnen. Der Winkel zwischen den beiden Drehachsen 18, 28 liegt vorzugsweise im Bereich von 0 bis 70°, besonders bevorzugt von 30 bis 70°.

Das "Rückdrehverhältnis" bezeichnet das Verhältnis der vollen Umdrehungen um die beiden Drehachsen 18, 28 einer DAZ zueinander. Es ist die Zahl der Umdrehungen, die der Hauptrotor 16 um die Drehachse 18 vollführen muß, damit sich der Probenbehälter 24 einmal um die Drehachse 28 gedreht hat. Das Rückdrehverhältnis wird entsprechend als XX:1 angegeben und ist positiv, wenn sich der Probenbehälter 24 entgegengesetzt zum Hauptdreharm 16 dreht.

Die Drehzahl des Auslegers 16 beträgt vorzugsweise wenigstens 200 Umdrehungen pro Minute (rpm) und maximal 4000 rpm. Sie liegt bevorzugt im Bereich von 2000-3540 rpm. Die Drehzahl des Behälters 24 um die Drehachse 28 ist vorzugsweise so eingestellt, dass sich ein Rückdrehverhältnis zwischen 1:6 und 6:1 ergibt. Sie beträgt vorzugsweise wenigstens ein Fünftel, besonders bevorzugt wenigstens ein Drittel der Drehzahl des Auslegers 16, was einem Rückdrehverhältnis von 5:1 bzw. 3:1 entspricht. Ferner sind die Drehrichtungen 20, 30 vorzugsweise einander entgegengesetzt.

Es können auch in einem geeigneten Halter Gefäße bzw. Mischbehälter in den Behälter 24 eingesetzt werden, insbesondere Injektionsfläschchen, Glasgefäße oder Kunststoffgefäße wie z. B. Eppendorf-Gefäße. Der Behälter 24 fungiert dann als Aufnahmebehälter. Das Verfahren nach Ausführungsform (1) erlaubt den Einsatz der unterschiedlichsten Gefäße. Bevorzugt sind Gefäße aus Glas oder Kunststoff, deren Durchmesser 5-75 mm, besonders bevorzugt 9-75 mm beträgt. Neben handelsüblichen Injektionsfläschchen sind speziell Eppendorfgefäße mit einem Durchmesser von 9 bis 10 mm und Glasgefäße mit einem Durchmesser von 9 bis 40 mm bevorzugt. Weitere geeignete Spezialgefäße zur Verwendung in Ausführungsform (1) sind die speziellen Mischbehälter, wie sie in Fig. 3 und 4 beispielhaft beschrieben sind. Auch die Verwendung von Tuben und Spritzen als Mischbehälter ist möglich.

Um das Mischen weiter zu verbessern, können innerhalb des Behälters 24 oder innerhalb der darin angeordneten Gefäße Mischhilfen (synonyme Begriffe: Dispergierhilfe, Homogenisierhilfe) vorgesehen sein. Hierbei kann es sich um eine definierte Rauheit der Innenseite des Behälters 24/des Gefäßes handeln. Ebenso können stegförmige Mischhilfen innerhalb des Behälters 24/des Gefäßes angeordnet sein. Bevorzugt sind Gefäße, deren Innenfläche aufgerauht, genoppt oder geriffelt ist. Mischhilfe können des weiteren Glasperlen in verschiedenen Mengen und Größen sein, Flügel an der inneren Gefäßwand oder ein entsprechend geformter zentraler und/oder peripherer Gefäßeinsatz. Besonders bevorzugt als Mischhilfe sind Perlen aus Materialien wie Edelstahl, Achat, Zirkonoxid, Wolframcarbid, Teflon, Teflon mit Stahlkern, Polyamid, Sinterkorund, Siliziumnitrid und Glas. Ganz besonders bevorzugt sind Glasperlen und Stahlperlen, vor allem Glasperlen. Bevorzugter Durchmesser dieser Perlen ist ein Durchmesser von mindestens 0,4 mm, besonders bevorzugt von 0,5 bis 6 mm.

"Speedmixen" bedeutet im Kontext der vorliegenden Erfindung die Nutzung eines Speedmixers^{®} oder einer anderen DAZ, bevorzugt eines Speedmixers^{®} vom Typ DAC 150 FVZ der Firma Hauschild GmbH und Co KG, welcher ein Rückdrehverhältnis von ca. 2,8:1 oder 4,2:1, besonders bevorzugt 4,2:1 aufweist, oder einer dualen asymmetrischen Zentrifuge, welche ein ähnliches Rückdrehverhältnis aufweist.

In der erfindungsgemäßen Verwendung (1) und dem erfindungsgemäßen Verfahren (3) sind Ansatzgrößen von wenigen Milligramm bis zu 3000 g (3 kg) möglich, bevorzugt sind Ansatzgrößen von 5 mg bis 150 g, ganz besonders bevorzugt von 20 mg bis 40 g.

Die Verwendung nach (1) bzw. das Verfahren nach (3) erfolgt bevorzugt mit einer DAZ, die zur Aufnahme von Reaktionsgefäßen der Größe von 0,1 bis 110 ml, bevorzugt von 0,1 bis 25 ml, besonders bevorzugt von 0,5 bis 10 ml, ganz besonders bevorzugt von 0,5 bis 2 ml geeignet ist.

Auf die zu mischenden, in dem Behälter 24 angeordneten Substanzen wird in der Verwendung nach (1) und dem Verfahren nach (3) vorzugsweise eine Zentrifugalkraft von wenigstens 1,2 g, bevorzugt wenigstens 80 g, besonders bevorzugt wenigstens 300 g, ganz besonders bevorzugt von 550 bis 1000 g oder von 620 bis 1500 g ausgeübt. Die maximale Zentrifugalkraft kann jedoch bis zu 3000 g betragen, bevorzugt bis zu 2500 g.

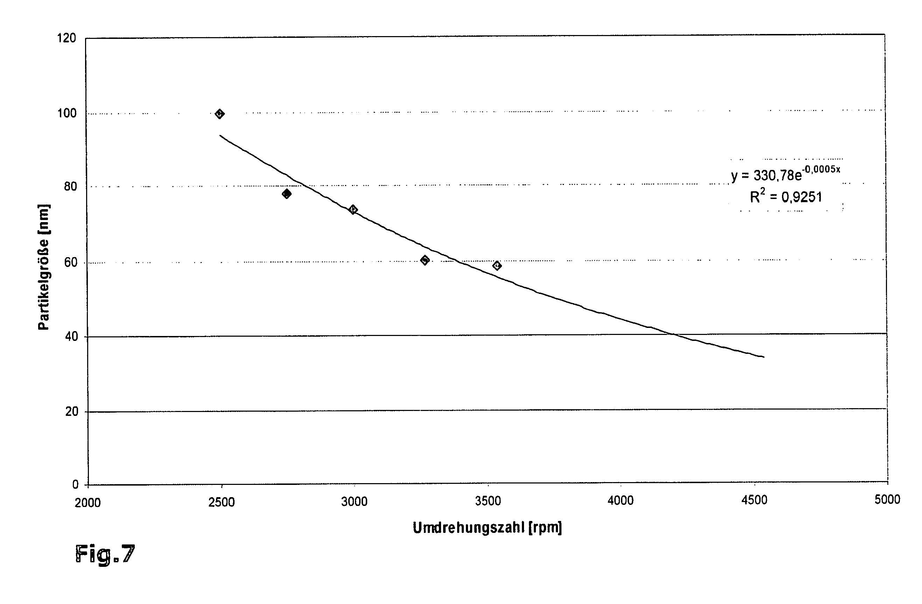

Die Vesikelgröße der erhaltenen Partikel hängt unter anderem von der Geschwindigkeit der DAZ ab. Je höher die Geschwindigkeit (in rpm) bzw. die g-Zahl, desto kleinere Partikelgrößen sind erreichbar (vgl. Bsp. 19). Relevante Geschwindigkeiten für die Herstellung insbesondere von kleinen Partikeln mit einem Durchmesser von unter 100 nm liegen im Bereich über 2000 rpm. Bei weiterer Steigerung der Geschwindigkeit sollten Teilchengrößen im Bereich von 30 nm Durchmesser realisierbar sein, voraussichtlich bei einer Geschwindigkeit von 4000 bis 5000 rpm (Fig. 7).

Die Homogenisierung im Verfahren der Ausführungsform (3) erfolgt in einem besonders bevorzugten Aspekt bei 3540 rpm und/oder bei 550 bis 1000 g. Das Rückdrehverhältnis im Verfahren nach (2) ist in einem besonders bevorzugten Aspekt kleiner oder gleich 4,2:1, ganz besonders bevorzugt kleiner oder gleich 2,8:1, die Zentrifugationszeit beträgt von 30 s bis 1 h, bevorzugt von 1 bis 40 min, besonders bevorzugt von 3 bis 30 min, ganz besonders bevorzugt von 3 bis 20 min.

Ein möglicher Aspekt der Ausführungsformen (1) und (3) ist der Einsatz eines Speedmixers^{®} als duale asymmetrische Zentrifuge, besonders bevorzugt eines Speedmixers^{®} vom Typ DAC 150 FVZ der Firma Hauschild GmbH und Co KG, welcher ein Rückdrehverhältnis von ca. 2,8:1 oder 4,2:1, bevorzugt von 4,2:1 aufweist.

Die erfindungsgemäße Verwendung nach (1) und das erfindungsgemäße Verfahren nach (3) ist hinsichtlich der Verwendung einer Mischhilfe, der Zentrifugiergeschwindigkeit, des Durchmessers des verwendeten Gefäßes, der Zentrifugierzeit und der Temperatur variierbar und eignet sich somit zur gezielten Herstellung von lipidbasierten Nanopartikeln in verschiedenen Partikelgrößen und Mengen (vgl. Bsp. 1 und 2). Bevorzugt haben die Nanopartikel eine Partikelgröße von 5 bis 1000 nm, besonders bevorzugt von 15 bis 200 nm, ganz besonders bevorzugt von 20 bis 150 nm. Wenn ein Nanopartikel definitionsgemäß keine Größe von 1000 nm haben darf, so bedeutet "bis 1000 nm" in diesem Kontext "bis zu 1000 nm", also kleiner als 1000 nm. Durch Variation der variablen Parameter können je nach Wunsch aus ein und derselben Ausgangslipidmischung ganz gezielt unterschiedliche liposomale Formulierungen hergestellt werden. Zudem ist es möglich, auch in Gefäßen mit geringem Durchmesser zu speedmixen, so dass auch Laborstandard-Gefäße wie Eppendorfgefäße^{®} etc. für ein Screening oder für Aufgaben im Bereich der Molekularbiologie eingesetzt werden können. Insbesondere bei einer Erhöhung der DAZ-Zentrifugiergeschwindigkeit und/oder Verkleinerung des Rückdrehverhältnisses ist bei kleinen Gefäßdurchmessern eine wesentliche Reduktion der Vesikelgrössen und eine Verringerung der notwendigen Zentrifugierzeit zu erwarten.

In der Verwendung (1) der Erfindung wird eine Mischvorrichtung zum Mischen von chemischen und/oder biologischen Substanzen eingesetzt. Bei der Mischvorrichtung erfolgt ein Mischen der Substanzen, insbesondere durch eine Bewegung eines Mischbehälters, die einem Schütteln entspricht. Die Mischvorrichtung weist einen Mischbehälter zur Aufnahme der zu mischenden Substanzen auf. Der Mischbehälter ist in einem Abstand zu einer ersten Drehachse vorgesehen und mit einem Ausleger verbunden. Der Ausleger ist mit einer ersten Antriebseinrichtung, wie einem Elektromotor verbunden, so dass der Ausleger um die erste Drehachse gedreht wird. Durch Drehen des Auslegers erfolgt somit ein Bewegen des Mischbehälters auf einer Kreislinie. Ferner ist eine zweite Antriebseinrichtung zum Drehen des Mischbehälters um eine zweite Drehachse vorgesehen. Die zweite Drehachse verläuft in einem Abstand zur ersten Drehachse. Insbesondere verläuft die zweite Drehachse durch den Mischbehälter. Erfindungsgemäße sind Innenwände des Mischbehälters in unterschiedlichen Abständen zu der zweiten Drehachse angeordnet. Dies hat bei einem Drehen des Mischbehälters um die erste Drehachse sowie um die zweite Drehachse zur Folge, dass während des Drehens unterschiedliche sich ändernde Kräfte auf die Substanzen wirken. Diese sind vergleichbar mit einer bei einer Schüttelbewegung auf Substanzen wirkende Kräfte. Hierdurch erfolgt ein besonders gutes homogenes Durchmischen der in dem Mischbehälter vorhandenen Substanzen.

Vorzugsweise handelt es sich bei dem Mischbehälter um einen zylindrischen Behälter. Die Längsachse des Mischbehälters ist hierbei vorzugsweise in einem Winkel zur zweiten Drehachse angeordnet. Der Winkel ist ungleich 0°. Hierdurch kann das Mischverhalten der Substanzen weiter verbessert werden. Vorzugsweise liegt der Winkel in einem Bereich von 70 bis 110°.

Bei einer besonders bevorzugten Ausführungsform ist der Mischbehälter in einem Aufnahmebehälter angeordnet. Hierbei kann es sich bei dem Aufnahmebehälter um den Behälter 24 der in dieser Anmeldung beschriebenen asymmetrischen Zentrifuge handeln. Es ist somit erfindungsgemäß möglich, einen beispielsweise zylindrischen Mischbehälter, beispielsweise liegend oder schräg in einem vorzugsweise ebenfalls zylindrischen Aufnahmebehälter anzuordnen. Hierzu ist vorzugsweise innerhalb des Aufnahmebehälters eine Halteeinrichtung zum lagegenauen Halten des Mischbehälters in dem Aufnahmebehälter vorgesehen.

In einer besonders bevorzugten Ausführungsform handelt es sich bei der Mischvorrichtung somit um eine Weiterentwicklung einer bekannten dualen asymmetrischen Zentrifuge, wie sie beispielsweise in EP 1 293 245 beschrieben ist. Bevorzugt ist die Mischvorrichtung, wie in Fig. 3 aufgebaut: Ein Mischbehälter 40 ist innerhalb des Aufnahmebehälters 24 angeordnet. Bei dem Aufnahmebehälter 24 handelt es sich um den an Hand der Fig. 1 und 2 beschriebenen mit dem Ausleger 16 verbundenen Behälter 24 einer asymmetrischen Zentrifuge.

Der Mischbehälter 40 ist im Wesentlichen zylindrisch ausgebildet und mittig in dem Aufnahmebehälter 24 angeordnet. Hierbei verläuft die zweite Drehachse 28 durch den Mischbehälter 40. Insbesondere verläuft die Drehachse 28 im Wesentlichen durch den Schwerpunkt des Mischbehälters 40 bzw. nahe des Schwerpunktes. Der Mischbehälter 40 ist mit einem Deckel 42 verschlossen. Um die Lage des Mischbehälters 40 in dem Aufnahmebehälter 24 eindeutig zu definieren, ist innerhalb des Aufnahmebehälters 24 eine nicht näher dargestellte Halteeinrichtung 44 vorgesehen. Bei dieser kann es sich beispielsweise um einen zylindrischen Körper aus Schaumstoff oder dgl. handeln, der eine Ausnehmung aufweist, in die der Mischbehälter 40 eingelegt werden kann.

Der Mischbehälter 40 ist im wesentlichen zylindrisch. Bevorzugt ist hierbei, dass der Querschnitt (senkrecht zum Zylindermantel) des Mischbehälters kreisförmig, elliptisch, oval oder dergleichen ist. Wesentlich ist hierbei, dass der Zylindermantel, bevorzugt der gesamte Mischbehälter 40 keine Ecken aufweist, da sich in solchen Ecken ungewollt Probenmaterial ansammeln kann. Vielmehr sind die Innenwände des Mischbehälters erfindungsgemäß bevorzugt rund ausgebildet bzw. mit Rundungen versehen.

Um ein gutes Durchmischen der Substanzen in dem Mischbehälter 40 zu gewährleisten, weist eine Innenwand 46, bei der es sich im dargestellten Ausführungsbeispiel um die Zylinderinnenwand handelt, zu der zweiten Drehachse 28 unterschiedliche Abstände auf. Diese sind als Beispiel durch gestrichelte Linien a und b angedeutet.

Durch Drehen des Aufnahmebehälters 24, wie an Hand der Fig. 1 und 2 beschrieben, wirken sich ändernde Kräfte auf die in dem Mischbehälter 40 angeordneten Substanzen.

Bei einer Ausführungsform der Verwendung (1) wird ein Mischbehälter in Verbindung mit der beschriebenen dualen asymmetrischen Zentrifuge verwendet, bei dem ein erster und ein zweiter Aufnahmeraum vorgesehen ist. Die Aufnahmeräume dienen zur Aufnahme einer ersten bzw. einer zweiten Substanz. Erfingdungsgemäß sind die beiden Aufnahmeräume durch eine Trennwand voneinander getrennt, wobei in der Trennwand eine Öffnung vorgesehen ist. Bevorzugt ist nur eine Öffnung, nicht jedoch mehrere Öffnungen (wie z.B. in einem Sieb) vorgesehen. Die Öffnung dient dazu, dass beim Auftreten von Zentrifugalkräften eine Substanz durch die Öffnung von einem Aufnahmeraum in den anderen Aufnahmeraum überführt wird. Hierbei ist die Querschnittsfläche der Öffnung vorzugsweise derart gewählt, dass in Abhängigkeit der Viskosität und der auftretenden Zentrifugalkräfte erst bei Zentrifugalkräften von mehr als 1,2 g, vorzugsweise mehr als 1,5 g ein Hindurchtreten der Substanz durch die Öffnung erfolgt. Vorzugsweise ist die Öffnung in einer Längsachse des ersten und/ oder des zweiten Aufnahmeraums angeordnet. Hierbei erfolgt sodann vorzugsweise ein Anordnen des erfindungsgemäßen Mischbehälters in einer Mischvorrichtung oder einer dualen asymmetrischen Zentrifuge derart, dass die Zentrifugalkräfte im Wesentlichen in Richtung der Längsachse wirken, d. h. dass sie ein Durchtreten des Substrates durch die Öffnung bewirken.

Vorzugsweise ist der zweite Aufnahmeraum innerhalb des ersten Aufnahmeraums angeordnet und insbesondere kegelförmig ausgebildet. Hierbei ist es besonders bevorzugt, dass die Öffnung an der Kegelspitze angeordnet ist. Bei dem ersten Aufnahmehohlraum kann es sich um einen zylindrischen Körper handeln. Der Mischbehälter ist vorzugsweise zu einer Längsachse rotationssymmetrisch ausgebildet. Die Längsachse verläuft vorzugsweise ein einem Winkel von etwa 90° zur zweiten Drehachse.

Ferner ist es möglich, dass mehrere zweite Aufnahmeräume vorgesehen sind und/oder der zweite Aufnahmeraum durch Trennwände in mehrere einzelne Räume bzw. Kammern unterteilt ist. Hierbei können zwischen den einzelnen Kammern und/oder Aufnahmeräumen und dem ersten Aufnahmeraum jeweils eine oder mehrere Öffnungen vorgesehen sein. Die Größe der Öffnungen ist jeweils vorzugsweise in Abhängigkeit der Viskosität der Flüssigkeit gewählt. Vorzugsweise ist die Ausrichtung der Öffnungen derart gewählt, dass die Zentrifugalkräfte auf die Öffnungen wirken.

Eine bevorzugte Ausführungsform des Mischbehälters ist in Fig. 4 dargestellt. Der in Fig. 4 dargestellte Mischbehälter 48 kann in dem Aufnahmebehälter 24 angeordnet werden. Das Mischen der Substanzen erfolgt sodann wie vorstehend an Hand der Fig. 1 und 2 beschrieben.

Der Mischbehälter 48 weist einen ersten Aufnahmeraum 50 und einen zweiten Aufnahmeraum 52 für eine erste bzw. eine zweite Substanz auf. Der erste Aufnahmeraum 50 ist durch einen zylindrischen Körper 54 gebildet. Der Körper 54 ist rotationssymmetrisch zu einer Längsachse 56.

Innerhalb des ersten Aufnahmeraums 50 ist zur Ausbildung des zweiten Aufnahmeraums 52 ein kegelförmiger Körper 58 angeordnet. Dieser ist ebenfalls rotationssymmetrisch zu der Längsachse 56 ausgebildet. Der Körper 58 ist in eine Öffnung 60 des zylindrischen Körpers 54 eingesteckt und wird an einem Rand 62 der Öffnung gehalten. Die Öffnung ist mit einem Deckel 64 verschlossen.

An einer Kegelspitze 66 des kegelförmigen Teils 58 ist eine Öffnung 68 vorgesehen. Die Öffnung 68 ist somit in einer Trennwand 70, durch die der erste Aufnahmeraum 50 von dem zweiten Aufnahmeraum 52 getrennt ist, vorgesehen.

Wird der Mischbehälter 48 nunmehr wie vorstehend an Hand der Fig. 1 und 2 beschrieben sowohl um eine erste Drehachse 18 als auch um eine zweite Drehachse 28 gedreht, wirken Zentrifugalkräfte auf die in der zweiten Kammer 52 vorgesehene Substanz. Hierdurch wird die Substanz durch die Öffnung 68 in den ersten Aufnahmeraum gedrückt bzw. übergeführt. Auf Grund der kegelstumpfförmigen Ausgestaltung ist gewährleistet, dass seitlich neben dem zweiten Aufnahmeraum in einem ringförmigen Bereich 72 Substrat aufgenommen werden kann, wenn die Zentrifugalkräfte von der Öffnung 68 in den zweiten Aufnahmeraum 52 hineingerichtet sind. Es ist hierdurch vermieden, dass größere Mengen an Substanzen aus dem ersten Aufnahmeraum 50 in den zweiten Aufnahmeraum 52 gelangen. Die Ausbildung eines insbesondere ringförmigen Raums 72 ist auf Grund der kegelförmigen Ausgestaltung des Teils 58 realisiert. Der zweite Aufnahmeraum 52 könnte jedoch auch eine andere Form aufweisen, wobei es bevorzugt ist, dass der zweite Aufnahmeraum 52 von einem Raum 72 umgeben ist, um ein Zurückdrücken von Substanzen aus dem ersten Aufnahmeraum 50 in den zweiten Aufnahmeraum 52 zu vermeiden.

Der zweite Aufnahmeraum 52 kann in zwei oder mehr Kammern bzw. Räume unterteilt sein. Vorzugsweise ist der zweite Aufnahmeraum 52 in zwei Kammern geteilt. Hierbei ist es bevorzugt, dass jede der Kammern eine Öffnung aufweist, die entsprechend der Öffnung 68 angeordnet ist und den gleichen Querschnitt aufweist. Je nach Anwendungsfall, insbesondere in Abhängigkeit der Viskosität der in den Kammern bzw. Räumen vorgesehenen Flüssigkeit, können jedoch auch andere Querschnitte der Öffnungen gewählt werden.

Der Mischbehälter 48 wird bevorzugt verwendet, wenn partikelhaltige Formulierungen redispergiert werden sollen (vgl. Bsp. 9C). In diesem Fall wird die partikelhaltige Formulierung im ersten Aufnahmeraum 50 vorgelegt und das Redispersionsmedium in den zweiten Aufnahmeraum 52 gegeben. Anschließend wird der Mischbehälter 48 wie oben beschrieben zentrifugiert. Er wird des Weiteren bevorzugt verwendet, wenn zwei Phasen miteinander vermischt werden sollen, um Mischungen oder lipidhaltige Nanopartikel zu erhalten (Bsp. 11). Diese Verwendung erlaubt auch den Einsatz eines Lösungsmittels in der lipidischen Phase (Bsp. 11C). Des Weiteren wird der Mischbehälter 48 bevorzugt bei Anwendung der Injektionsmethode und zur Herstellung von Lipoplexen verwendet. Mit anderen Worten: der Mischbehälter ist bevorzugt für Mischvorgänge zu verwenden.

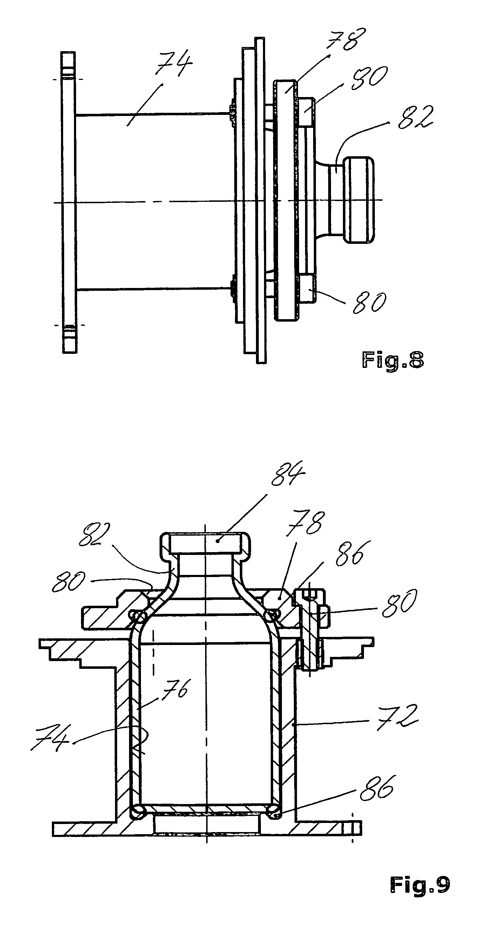

In den Aufnahmebehälter 24 kann in einer weiteren bevorzugten Ausführungsform der Erfindung ein Einsatz eingesetzt werden. Ein erster Einsatz, der insbesondere zur Aufnahme einer Injektionsflasche dient, ist in den Fig. 8 und 9 dargestellt (Ausführungsform (13)). Der Einsatz 72 weist einen Aufnahmeraum 74 auf, der im dargestellten Ausführungsbeispiel zylindrisch ist. Der Einsatz 72 kann in dem Aufnahmebehälter durch Rastelemente oder andere Befestigungsmittel befestigt werden. In den zylindrischen Aufnahmeraum ist ein Mischbehälter 76, bei dem es sich insbesondere um eine Injektionsflasche handeln kann, einsteckbar. Hierbei entsprechen die Innenabmessungen des Aufnahmeraums den Außenabmessungen der Injektionsflasche 76. Vorzugsweise liegt die Außenwand des Mischbehälters 76 vollständig an der Innenwand des Aufnahmeraums 74 an. Der Aufnahmeraum 74 kann jedoch auch mehrere Stege aufweisen, an denen die Außenseite des Mischbehälters 76 anliegt. Insbesondere sind drei Stege vorgesehen, die um 120 Grad zueinander versetzt sind.

Zum Halten des Mischbehälters 76 in dem Aufnahmeraum 74 ist ein Deckel 78 vorgesehen. Der Deckel 78 ist über Schrauben 80 oder andere Befestigungsmittel mit dem Einsatz 72 verbunden. Der Deckel 78 weist eine Öffnung 80 auf, durch die ein Flaschenhals 82 der Injektionsflasche 76 geführt ist. Somit ist ein Verschluss 84 der Injektionsflasche 76 auch dann zugänglich, wenn die Injektionsflasche 76 in dem Einsatz 72 eingesetzt ist.

Um ein Beschädigen einer insbesondere aus Glas hergestellten Injektionsflasche zu vermeiden, sind im dargestellten Ausführungsbeispiel ringförmige Dämpfungselemente 86 vorgesehen. Bei den Dämpfungselementen handelt es sich insbesondere um elastomere Ringe. Hierbei ist ein Dämpfungselement im Bodenbereich des Aufnahmeraums 74 und ein Dämpfungselement 86 im Deckel 78 angeordnet. Wie insbesondere aus Fig. 9 ersichtlich, ist durch die Dämpfungselemente beim Verschließen des Deckels 78 über die Schrauben 80 ein Beschädigen der Injektionsflasche 76 vermieden. Die Dämpfungselemente 86 sind hierzu an den entsprechend kritischen Stellen, insbesondere den Krafteinleitungsstellen am Deckel angeordnet. Die Dämpfungselemente dienen insbesondere zur Fixierung der Flasche im Einsatz. So wird eine Rückdrehung der Flasche infolge der wirkenden Zentrifugalkräfte unterbunden.

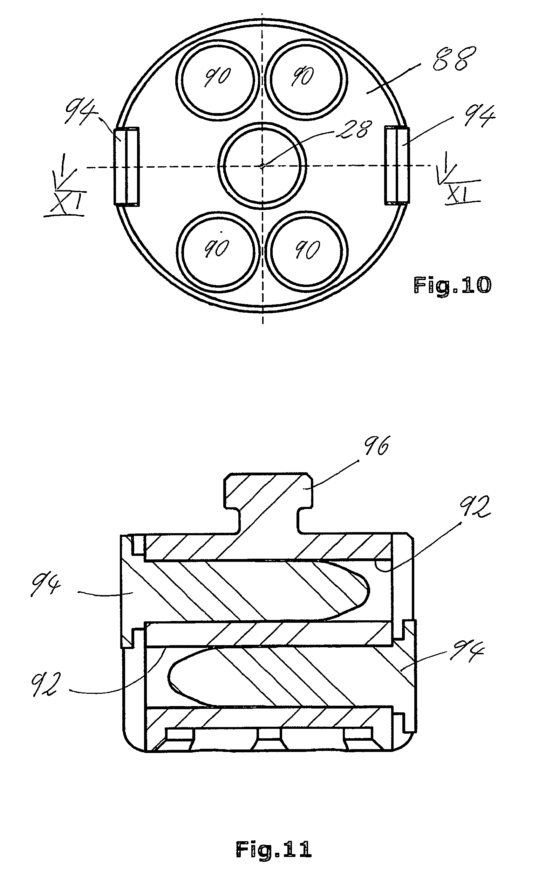

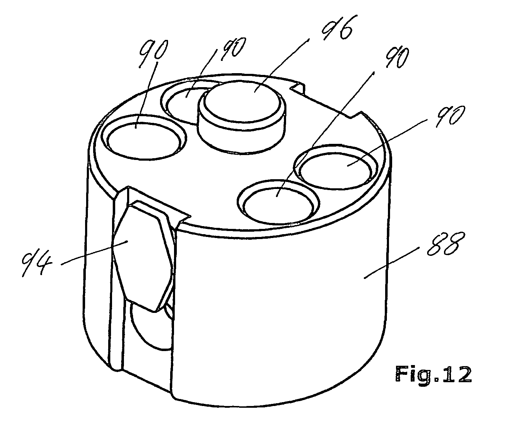

Zur Aufnahme von Mischbehältern 40 (Fig. 3), Mischbehältern 48 (Fig. 4), von Kryo-Vials oder Eppendorfgefäßen und dergleichen, kann ein Aufnahmebehälter-einsatz 88 (Fig. 10 bis 12) vorgesehen sein. Der Aufnahmebehälter 88 weist im dargestellten Ausführungsbeispiel sechs Aufnahmeräume 90, 92 auf. Im dargestellten Ausführungsbeispiel sind die Aufnahmeräume 90, 92 kreiszylindrisch ausgebildet. Die Aufnahmeräume können jedoch auch beispielsweise durch das Vorsehen von Stegen gebildet sein, die zur Lagefixierung der Mischbehälter dienen. Hierbei können sämtliche oder zumindest einzelne Stege elastisch ausgebildet sein.

Die Aufnahmeräume 90 sind im dargestellten Ausführungsbeispiel parallel zur zweiten Drehachse 28 angeordnet. Die Längsachse der Aufnahmeräume 90 verläuft somit parallel zur Drehachse 28 bzw. senkrecht zur Zeichenebene der Fig. 10. Die Längsachsen der Aufnahmeräume 90 sind in einem Abstand zur zweiten Drehachse 28 angeordnet. Die zweiten Aufnahmeräume 92 (Fig. 11) sind senkrecht zur zweiten Drehachse 28 ausgerichtet.

In dem dargestellten Ausführungsbeispiel sind in die Aufnahmeräume 92 Eppendorfgefäße 94 eingesteckt. Hierbei sind die Aufnahmeräume 92 vorzugsweise als Durchgangsöffnungen in dem Einsatz 88 ausgebildet, so dass die Eppendorfgefäße 94 von unterschiedlichen Seiten in die Aufnahmeräume 92 eingesteckt werden können.

Zum erleichterten Transport weist der Einsatz 88 ein Griffelement 96 auf. Das Griffelement 96 ist insbesondere auch zum Einführen und Herausnehmen des Einsatzes in den Aufnahmebehälter 24 geeignet.

Eine vorstehend beschriebene Mischvorrichtung ist geeignet, um mit Hilfe einer DAZ auch Schüttelaufgaben zu lösen. Für die Mischvorrichtung wurde ein DAZ-Einsatz entwickelt, bei dem die Vials horizontal in den Probenhalter eingebracht werden (siehe Fig. 3 und 10 bis 12). Mit einem solchen Einsatz ist praktisch jede Laborschüttelaufgabe sehr effizient, leise und rasch zu leisten.

Das hier entwickelte Verfahren ist erheblich besser als gängige Verfahren (wie z.B. FastPrep^{®} von Qbiogene), weil z. B. zwischen den einzelnen Schüttelvorgängen keine 5-Minuten-Pause eingelegt werden muss und das hierfür benötigte Gerät (DAZ) auch für andere Aufgaben eingesetzt werden kann. Darüber hinaus ist das Schütteln in verschiedenen Gefäßen durch einfachen Austausch des Mischbehälters in der Mischvorrichtung gemäß (10) möglich. Bei Ausführung des DAZ-Einsatzes aus isolierendem Material wie Styropor bleiben tiefgekühlte Proben länger kalt, was insbesondere bei Gewebeaufschluss von Vorteil ist, da so eine schonende Behandlung des Materials trotz des of erforderlichen längeren Schüttelns der Probe gewährleistet ist. Insbesondere können mit dieser Technik auch sehr kleine Probenmengen zertrümmert werden. Hierzu wurden tieftemperatur-geeignete Aufnahmebehälter für Probenvials hergestellt (Fig. 10-12), in die verschiedene Typen von sog. Kryo-Vials und Eppendorf^{®}-Gefäßen fest eingespannt werden können. "Tieftemperatur-geeingnet" bedeutet, dass diese Behälter aus einem Material sind, das für die Verwendung bei Temperaturen bis minimal -196 °C bzw. in flüssigem Stickstoff geeignet ist. Das Material, aus welchem solche Aufnahmebehälter hergestellt werden, ist vorzugsweise Teflon, PE UHMW oder Aluminium. Für nicht-tieftemperaturgeeignete Aufnahmebehälter können auch andere Materialien verwendet werden, bevorzugt leichte Materialien wie Styropor und Kunststoff.

Eine DAZ, insbesondere eine DAZ mit den Vorrichtungen für das Schüttelverfahren kann also auch zur Zerkleinerung von Feststoffen, insbesondere für die Gewebeaufarbeitung und den Aufschluss von Zellen eingesetzt werden (Bsp. 17). Zerkleinerung bedeutet bevorzugt, dass die Feststoffe nach Durchführung des Verfahrens als Pulver vorliegen. Das entsprechenden Verfahren gemäß Ausführungsform (3) wird unter Zusatz von Zertrümmerungshilfen durchgeführt. Geeignete Zetrümmerungshilfen sind die oben beschriebenen Dispergierhilfen, also Perlen aus Materialien wie Stahl, Achat, Korund und Glas. Ganz besonders bevorzugt sind Stahl- oder Glasperlen. Ihr Durchmesser beträgt bevorzugt mindestens 3 mm, ganz besonders bevorzugt mindestens 5 mm. Der bevorzugte maximale Durchmesser ist 10 mm. Auch Mischungen aus verschiedenen Zertrümmerungshilfen (verschiedene Größe und/oder verschiedene Materialien) können verwendet werden. Wenn Gewebe mit diesem Verfahren zertrümmert werden, so sind sie tierischen (auch menschlichen) oder pflanzlichen Ursprungs. Aufzuschließende Zellen können entweder eukaryotisch oder prokaryotisch sein.

Der Aufschluss der Zellen bzw. Gewebe gemäß Ausführungsform (3) kann nicht nur bei Raumtemperatur, sondern auch bei tiefen Temperaturen durchgeführt werden. Hierzu wird die Probe im Probenbehälter gekühlt oder tiefgefroren, z.B. durch Zugabe von flüssigem Stickstoff, und der Aufschluss In der DAZ erfolgt in einem vorgekühlten tieftemperatur-geeigneten Aufnahmebehälter, wie er oben beschrieben wird (Bsp. 17, Fig. 10-12)

Das Verfahren nach Ausführungsform (3) kann auch verwendet werden, um andere Proben als Gewebe oder Zellen zu zerkleinern. Solche anderen Proben sind all jene Materialien, die durch Mörsern zerkleinert werden können. Bevorzugt sind dies Nahrungsmittel, feste Arzneiformen einschließlich Tabletten und Granulaten, und kristalline organische oder anorganische Materialien (vgl. Bsp. 17). Diese Zerkleinerung dient bevorzugt der Probenvorbereitung für die Analytik, wie z.B. der Vorbereitung von KBr-Presslingen für die Infrarotspektrometrie oder der Bestimmung des Wirkstoffgehalts eines Arzneimittels.

Das Verfahren nach Ausführungsform (3) ist gewissermaßen ein Ersatz für den oft mühsam zu handhabenden und für kleine Probenmengen eher ungeeigneten klassischen Mörser. Es ist bevorzugt für die Zertrümmerung kleiner Probenmengen (weniger als 1 g, insbesondere weniger als 300 mg) und für die Handhabung gefährlicher, giftiger oder radioaktiver Substanzen geeignet.

Ein weiterer Vorteil des Verfahrens (3) ist, dass es in Gefäßen mit geringen Volumina durchgeführt werden kann. Bevorzugt sind dies Volumina bis maximal 10 ml, besonders bevorzugt bis maximal 2 ml. Auch die Arbeit in noch kleineren Volumina ist möglich. Dies erlaubt die Arbeit mit kleinen biologischen Proben und das zeitsparende parallele Zerkleinern einer Vielzahl von Proben. Bevorzugte Gefäße sind Eppendorf^{®}-Gefäße und Kryo-Vials.

Die Erfindung wird anhand der nachfolgenden Beispiele näher erläutert. Beispiele, die nicht mehr unter den Umfang der Ansprüche fallen, dienen illustrativen Zwecken.

### Beispiele

In den folgenden Beispielen bedeutet "Speedmixen" die Nutzung eines Speedmixers^{®} (asymmetrische duale Zentrifuge) vom Typ DAC 150 FVZ der Firma Hauschild GmbH und Co KG, welcher ein Rückdrehverhältnis von ca. 4,2:1 aufweist. "Eppi" bedeutet ein 2 ml-Eppendorf^{®}-Gefäß. Soweit nicht anders angegeben, beziehen sich %-Angaben auf % (w/v). Soweit nicht anders angegeben, bedeutet "Größe" der lipidbasierten Nanopartikel im folgenden stets "mittlere Größe".

### Beispiel 1: Herstellung von vesikulären Phospholipid Gelen (VPG) und liposomalen Dispersionen mit Hilfe der DAZ-Technik; Vergleich der entstandenen Liposomen mit Liposomen, die mit Hilfe der Hochdruckhomogenisation hergestellt wurden

1,48 g einer als feste Lösung vorliegenden Mischung aus EPC3 (hydriertes Eilecithin, Fa. Lipoid) und Cholesterol (55 mol%:45 mol%) wurde in eine 25 ml Arzneimittelflasche (Durchmesser außen: 36 mm) eingewogen und zugebördelt (Vial 1). Eine zweite Arzneimittelflasche (Vial 2) enthielt die gleiche Menge Lipid, allerdings wurden hier zusätzlich 3,0 g Glasperlen (Durchmesser ca. 1 mm) zugegeben. In beide Vials wurden 2,22 ml Mannitollösung (5%) injiziert und die Vials jeweils 4*5min bei RT (Raumtemperatur) mit 3540 rpm gespeedmixt. Durch Zugabe von 6,4 ml Mannitollösung (5%) in 2 Schritten (Schritt 1 : 100µl) wurde das entstandene vesikuläre Phospholipidgel (VPG) redispergiert (je 1 min speedmixen) und die mittlere Größe der Liposomen durch Photonenkorrelationsspektroskopie (PCS, Nicomp 370) bestimmt. Die Anzahl der Teilchen > 1 µm wurde mit Hilfe des Abschattungsverfahrens bestimmt (Fa. Nicomp, Accusizer). In die Bestimmung wurde auch VPG einbezogen, welches durch Hochdruckhomogenisation (gleiche Lipidmischung, gleicher Anteil Mannitollösung; 700 bar, 10 Zyklen) hergestellt wurde (Vial 3). Ebenfalls mit einbezogen in die Untersuchung von Teilchen > 1 µm wurde eine kommerzielle Fettemulsion für die parenterale Ernährung (Nutriflex^{®}, Fa. B. Braun).

**Tabelle 1: Vergleich der Liposomengröße und der Zahl der Teilchen, die > 1 µm sind, nach Speedmixen ohne (vial 1) und mit (vial 2) Glasperlen, nach Hochdruckhomogenisation (vial 3) und in einer kommerziellen Fettemulsion (Nutriflex^{®}).**

| Vial | Größe der Liposomen | Anzahl Teilchen > 1 µm pro ml (Verdünnung: 1:20 Mio.) |
|---|---|---|
| 1 | 171 nm | 173 |
| 2 | 36 nm | 97 |
| 3 | 36 nm | 209 |
| Nutriflex^{®} | 190 nm | 58 |

### Beispiel 2: Herstellung von vesikulären Phospholipid Gelen (VPG) und liposomalen Dispersionen mit Hilfe der DAZ-Technik: Variation der DAZ-Parameter (i) Mix-Geschwindicikeit, (ii) Gefäßdurchmesser. (iii) Speedmix-Zeit, (iv) Temperatureinfluß und (v) Zusatz einer Mischhilfe

Bei den im folgenden beschriebenen Experimenten wurden Liposomen mit Hilfe der DAZ-Technik analog zu Beispiel 1 hergestellt (gleiche Lipidmischung (als feste Lösung vorliegenden Mischung aus EPC3 (hydriertes Eilecithin, Fa. Lipoid) und Cholesterol (55:45 mol%)), gleiches wässeriges Medium (5%ige Mannitollösung)). Variiert wurde die Geschwindigkeit des Speedmixers®, die Zeit des Speedmixens, der Durchmesser des für das Speedmixen eingesetzten Gefäßes und die Temperatur. Zudem wurde mit und ohne den Zusatz einer Mischhilfe gearbeitet, hierfür wurden bei diesen Beispielen Glasperlen eingesetzt.

Das beim Speedmixen entstandene Liposomen-Gel wurde mit den 3-fachen des primär eingesetzten wässerigen Volumens redispergiert und die jeweils entstandenen Liposomen wurden anschließend mit Hilfe der PCS auf ihre mittlere Größe untersucht.

**Tabelle 2: Effekte der Variation der Speedmix-Parameter**

| Experiment Nr. | Mix-Gesch windigkeit [rpm] | Temperatur | Gefäßtyp/ Durchmesser [mm] | Zeit [min] | Glasperlen [mg] | Ansatzgröße [mg Lipid] | Part ikelgröße [nm] (Bulkmenge) |
|---|---|---|---|---|---|---|---|
| Einfluss der Zeit | | | | | | | |
| 3a | 3540 | RT | Eppi/10 | 20 | 100 | 50 | 72,7 |
| 3b | 3540 | RT | Eppi/10 | 30 | 100 | 50 | 97,0 |
| 3c | 3540 | RT | Eppi/10 | 50 | 100 | 50 | 69,4 |
| 3d | 3540 | RT | Eppi/10 | 70 | 100 | 50 | 59,4 |
| 3e | 3540 | RT | Eppi/10 | 90 | 100 | 50 | 53,5 |

| Einfluss der Temperatur | | | | | | | |
|---|---|---|---|---|---|---|---|
| 11 | 3540 | 60 °C Heizblock Siliconöl | Eppi/10 | 20 | 100 | 50 | 55,3 |
| 17b | 3540 | RT | Glas/10 | 5 | 100 | 50 | 79,7 |
| 18a | 3540 | 60 Heizblock Siliconöl | Glas/10 | 5 | 100 | 50 | 40,3 |

| Einfluss Gefäß und Mischhilfe (Glasperlen) | | | | | | | |
|---|---|---|---|---|---|---|---|
| X17b | 3540 | RT | Glas/10 | 5 | 100 | 50 | 79,7 |
| 17c | 3540 | RT | Glas/11 | 5 | 100 | 50 | 87 |
| 17d | 3540 | RT | Glas/ 15 | 5 | 100 | 50 | 53,6 |
| 17f | 3540 | RT | Glas/10 | 5 | - | 50 | 87,4 |
| 17g | 3540 | RT | Glas/11 | 5 | - | 50 | 271 |
| 17h | 3540 | RT | Glas/15 | 5 | - | 50 | 119,5 |

| Einfluss der Mixgeschwindigkeit | | | | | | | |
|---|---|---|---|---|---|---|---|
| X17b | 3540 | RT | Glas/10 | 5 | 100 | 50 | 79,7 |
| 30a | 3000 | RT | Glas/10 | 5 | 100 | 50 | 285,1 |
| 30b | 2000 | RT | Glas/10 | 5 | 100 | 50 | Keine Liposomenformation |

X: Experiment ist aus Vergleichsgründen nochmals aufgeführt

### Beispiel 3: Herstellung von vesikulären Phospholipid Gelen (VPG) und/oder liposomalen Dispersionen aus verschiedenen Lipiden/Lipidmischungen mit Hilfe der DAZ-Technik

Bei den im folgenden beschriebenen Experimenten wurden Liposomen mit Hilfe der DAZ-Technik analog zu Beispiel 1 und 2 hergestellt. Variiert wurden bei diesem Experiment die Lipidmischungen. Tabelle 3 stellt die Experimente dar, bei denen Lipidmischungen eingesetzt wurden, die bereits als feste Lösungen vorlagen bzw. bei denen nur Lipide eines Typs eingesetzt wurden. Hierzu wurden die Lipidmischungen zuvor in CHCl₃/MeOH 2:1 aufgenommen, das Lösungsmittel abrotiert und der entstandene Lipidfilm im Vakuum getrocknet und aus dem Kolben gekratzt bzw. die Lipidmischung wurde schon molekulardispers geliefert.

Bei den Experimenten, die in Tabelle 4 dargestellt sind, wurden die Lipide als Einzelkomponenten eingesetzt. Zum Vergleich wurden Werte aus Tab.3 mit Lipidmischungen, die als feste Lösung verwendet wurden, nochmals aufgeführt (durch "X" bzw. "* " markiert).

Die beim Speedmixen entstandenen Liposomen-Gele wurde mit dem 3fachen des primär eingesetzten wässrigen Volumens redispergiert. Die jeweils entstandenen Liposomen wurden anschließend mit Hilfe der PCS auf ihre mittlere Größe untersucht.

Tabelle 3: Experimente zur Herstellung von Liposomen aus Lipidmischungen, die als feste Lösungen eingesetzt wurden

| Experiment Nr. | Lipid-Komposition | Ansatzgröße [mg Lipid] | Gefäßtyp/ Durchmesser [mm] | Zeit [min] | Glasperlen [mg] | Partikelgröße [nm] (Bulkmenge) |
|---|---|---|---|---|---|---|
| Hydriertes Ei-Phosphatidylcholin/Cholesterol (55/45 mol/mol): siehe Bsp. 2 | | | | | | |
| Phosphatidylcholin aus Soja (min. 80%, S80 Fa. Lipoid), 5% Mannitollösung | | | | | | |
| 4a | E80 | 50 | Eppi | 20 | 100 | 52,7 |
| 4b | E80 | 50 | Eppi | 30 | 100 | 53,5 |
| 4c | E80 | 50 | Eppi | 40 | 100 | 52,4 |
| 8 | E80 | 50 | Eppi | 20 | 100 | 47,3 |

| Kationische Lipide | | | | | | |
|---|---|---|---|---|---|---|
| 5a | DOTAP | 50 | Eppi | 20 | 100 | 57,7 |
| 31 | KL-1-14/Chol 1:0,55 | 50 | Eppi | 10 | 100 | 112,2 |

| Surfactant-Lipide (Aleofact, BI) | | | | | | |
|---|---|---|---|---|---|---|
| 21 | Surfactant-Lipide | Ca. 50 | Original- | 5 | - | 48,1 |

| Experiment Nr. | Lipid-Komposition | Ansatzgröße [mg Lipid] | Gefäßtyp/ Durchmesser [mm] | Zeit [min] | Glasperlen [mg] | Partikelgröße [nm] (Bulkmenge) |
|---|---|---|---|---|---|---|
| | | | Vial, Glas Durchmess er: 14mm | | | |

| Negativ geladene Lipidmischung | | | | | | |
|---|---|---|---|---|---|---|
| 32a | HEPC, Chol, DPPG | 50 | Eppi | 5 | 100 | 238,1 |

| Stealth-Liposomen | | | | | | |
|---|---|---|---|---|---|---|
| 29b | HePC/Chol/DSPE-PEG-2000 | 104 | Glas 11mm | 25 | 1200 | 88,4 |

**Tabelle 4: Experimente zur Herstellung von Liposomen aus Lipidmischungen, die als Einzelkomponenten in die Experimente eingesetzt wurden**

| Experiment No | Lipid-Komposition | Ansatzgröße [mg Lipid] | Gefäßtyp/ Durch messer [mm] | Zeit [min] | Glasperlen [mg] | Partikelgröße [nm] (Bulkmenge) | Zusatzbehandlung |
|---|---|---|---|---|---|---|---|
| Hydriertes Ei-Phosphatidylcholin/Cholesterol (55/45 mol%/mol%) | | | | | | | |
| 15a | EPC3/Chol | 800 | PE-Becher/14 | 20 | 1600 | 60,4 | |
| 15a | EPC3/ Chol | 800 | PE-Becher/14 | 25 | 1600 | 54,8 | |
| 15a | EPC3/ Chol | 800 | PE-Becher/14 | 30 | 1600 | 41,9 | |
| X2 Vergleich | EPC3/Chol-Fertigmischung von Lipoid | 1480 | Injektions-flasche/30 | 20 | 3000 | 36 | |
| 11 | EPC3/Chol | 50 | Eppi | 20 | 100 | Keine Liposomenformation | RT |
| 11a | EPC3/Chol | 50 | Eppi | 20 | 100 | 56,1 | Erhitzt auf 60°C |

| E80/Chol-Gemische (im Eppi, im großen PE-Becher mit und ohne Erwärmung) | | | | | | | |
|---|---|---|---|---|---|---|---|
| 9 | E80/Chol | 50 | Eppi | 30 | 100 | 200,9 | |
| 13a | E80/Chol | 1200 | PE-Becher/14 | 1,5 | 2400 | 133,7 | |
| 13b | E80/Chol | 1200 | PE-Becher/14 | 10 | 2400 | 66,3 | |
| 13c | E80/Chol | 1200 | PE-Becher/ 14 | 15 | 2400 | 55,3 | |
| 13d | E80/Chol | 1200 | PE-Becher/ 14 | 20 | 2400 | 71,2 | |
| 13e | E80/Chol | 1200 | PE-Becher/14 | 25 | 2400 | 70,4 | |
| 14a | E80/Chol | 1200 | PE-Becher/14 | 1 | 2400 | 158,3 | Erhitzt auf 60°C |
| 14b | E80/Chol | 1200 | PE-Becher/14 | 5 | 2400 | 85,7 | Erhitzt auf 60 °C |
| 14c | E80/Chol | 1200 | PE-Becher/14 | 10 | 2400 | 65,4 | Erhitzt au f 60 °C |
| 14d | E80/Chol | 1200 | PE-Becher/14 | 15 | 2400 | 60,2 | Erhitzt auf 60 °C |
| 14e | E80/Chol | 1200 | PE- | 20 | 2400 | 54 | Erhitzt |

| Experiment No | Lipid-Komposition | Ansatzgröße [mg Lipid] | Gefäßtyp/ Durchmesser [mm] | Zeit [min] | Glasperlen [mg] | Partikelgröße [nm] (Bulkmenge) | Zusatzbehandlung |
|---|---|---|---|---|---|---|---|
| | | | Becher/ 14 | | | | auf 60°C |

| Kationisches Lipid KL-1-14/Chol (Variation: Mix-Zeit und Temperatur) | | | | | | | |
|---|---|---|---|---|---|---|---|
| 26a | KL-1-14/Chol | 50 | Eppi | 20 | 100 | 371,2 | |
| 26b | KL-1-14/Chol | 50 | Eppi | 30 | 100 | 298,9 | |
| 26c | KL-1-14/Chol | 50 | Eppi | 40 | 100 | 299,9 | |
| 26d | KL-1-14/Chol | 50 | Eppi | 50 | 100 | 197,7 | Erhitzt auf 60°C |
| X 31 | KL-1-14/Chol* 1:0,55 | 50 | Eppi | 10 | 100 | 112,2 | |

| Stealth-Liposomen | | | | | | | |
|---|---|---|---|---|---|---|---|
| 29a | HePC/Chol/DSPE-PEG-2000 | 114 | Glas/10 | 25 | 1500 | 70,3 | |
| X 29b | HePC/Chol/DSPE-PEG-2000* | 114 | Glas/10 | 25 | 1500 | 88,4 | |

| | | | | | | | |
|---|---|---|---|---|---|---|---|
| X: Vergleichsexperimente mit Lipidmischungen, die als feste Lösung eingesetzt wurden; * : feste Lösung von Lipidmischungen | | | | | | | |

### Beispiel 4: Herstellung von Surfactant-ähnlichen Liposomen, die für therapeutische Zwecke in die Lunge eingebracht werden können

A) Surfactant-ähnliche Liposomen ohne spezielle Wirkstoffe. (Komposition nach Wissel et al., Am J Physiol 271: L432-40 (1996); oder Müller, B. et al., Thorax 58:127-134 (2003); oder al-Mehdi, A.B. et al., BBA 1167:56-62 (1993)). Die Liposomen wurden in Analogie zum oben beschriebenen Vorgehen durch Speedmixen hergestellt. Alle Arbeiten wurden in einer Sterilbank durchgeführt. Die Lipidkomposition war DPPC (Sigma P5911)/PG (Sigma-P05141) /EPC (Lipoid) /Cholesterol im Gewichtsverhältnis 5,5/1/2,5/1. Die Lipide wurden eingewogen, in Chloroform/MeOH 2:1 aufgenommen, das Lösemittel abrotiert und der entstandene Lipidfilm getrocknet. 50 mg dieses Lipidgemisches wurde dann in ein autoklaviertes Speedmix-Gefäß (Glasvial, 10 mm) eingewogen, 100 mg autoklavierte Glasperlen zugefügt und ein Volumen von 75 µl sterilfiltriertem PBS (25 mM NaH₂PO₄, 125 mM NaCl, pH 6,9) zugegeben und für 10 Minuten im geschlossen Gefäß gespeedmixt. Anschließend wurde mit 250 µl PBS redispergiert. Es wurden Liposomen der Größe 74,6 nm erhalten.
B) Surfactant-ähnliche Liposomen mit Fluoreszenz- (NBD: Rhodamin) und Radio-Label (³H). Für dieses Experiment wurden folgende Lipide in ein steriles Glasgefäß eingewogen bzw. als Lösung in CHCl₃/MeOH 2:1 zugegeben: DPPC, 81,2 mg (Sigma P5911) / PG, 17,1 mg (Sigma-P05141) / EPC, 42,7 mg (Lipoid) / Cholesterol, 17,1 mg / Rhodamin-PE, 1,7 mg / NBD-PC 7,7 mg / ³H-DPPC (1,46nCi/µg DPPC). Die Mischung wurde im Speedmixgefäß (Glas 15 mm) vollständig mit CHCl₃/MeOH 2:1 gelöst und das Lösungsmittel vorsichtig in einem Stickstoffstrom bis zur Trockene abgedampft. 250 µl steriler PBS-Puffer und 350 mg sterile Glasperlen (Durchmesser 1 mm) wurden zugegeben und die Mischung im geschlossenen Gefäß für 15 Minuten gespeedmixt. Anschließend wurde 17,2 ml sterile PBS-Lösung zugegeben und nochmals für 1 Minute gespeedmixt. Die Vesikel der fertigen liposomalen Dispersion hatten eine mittlere Größe von 83,5 nm.
C) Surfactant-ähnliche Liposomen mit einem lipidischen sPLA2-Inhibitor als antiinflammatorischem Wirkstoff. DPPC, PG, EPC, Cholesterol und der sPLA2-Inhibitor 2-(R)-1-O-Phosphocholin-2-N-laurinoyl-octadecan in den Gewichtsverhältnissen 50/10/25/10/5 wurden gemeinsam eingewogen und in CHCl₃/Methanol 2:1 gelöst. Das Lösungsmittel wurde abrotiert, der entstandene Lipidfilm im Vakuum von Lösemittelresten befreit und die Lipidmischung aus dem Kolben gekratzt. Eine Menge von 100 mg Lipidmischung wurde in ein steriles Glasgefäß eingewogen, 200 mg sterile Glasperlen (Durchmesser 1 mm) zugegeben und 150 µl PBS-Puffer (10 mM NaH₂PO₄, 149 mM NaCl, pH 6,9) zugesetzt. Das Gemisch wurde 15 Minuten gespeedmixt, anschließend wurde das entstandene Gel mit 9,8 ml PBS redispergiert. Die Vesikel der fertigen Dispersion hatten eine mittlere Größe von 74,3 nm.

### Beispiel 5: Direkte Herstellung von mit Substanzen beladenen vesikulären Phospholipid Gelen (VPG) und liposomalen Dispersionen mit Hilfe der DAZ-Technik

A) Direkter Einschluss von Albumin in Liposomen. In sechs sterile Glasvials (Durchmesser 10 mm) wurden je 100 mg Glasperlen (Durchmesser 1 mm) sowie jeweils 50 mg der als feste Lösung vorliegenden Mischung von hydriertem Ei-Phosphatidylcholin und Cholesterol (55/45 mol%) eingewogen. In drei der Vials wurde je 75 µl einer Albuminlösung in den Konzentrationen 1, 2 und 3 mg/ml zupipettiert. In das vierte bis sechste Vial wurden je 75 µl NaCl-Lösung der Konzentration 0,9 % gegeben. Die verschlossenen Vials wurden je 10 Minuten bei 3540 rpm gespeedmixt. Die drei mit Albumin beladenen Liposomen-Gele wurden mit je 250 µl NaCl-Lösung redispergiert. Die nur mit Kochsalzlösung hergestellten Liposomen-Gele (Vial 4-6) wurden hingegen mit 175 µl NaCl-Lösung plus 75 µl Albuminlösung (1, 2, 3 mg/ml) redispergiert. Anschließend wurden die so entstandenen liposomalen Dispersionen in geeignete Zentrifugenröhrchen pipettiert und bei 350.000 g zentrifugiert, wobei die Liposomen sedimentierten. Im Überstand wurde anschließend die Albuminkonzentration mit Hilfe eines BCA-Tests (Interchim/ Uptima, Nr. UP95424) bestimmt und die Einschlusseffizienz berechnet. Ergebnis Einschlusseffizienz für Albumin in Liposomen durch Speedmixen: 1 mg/ml Albumin: 60,9 %; 2 mg/ml Albumin: 70,9 %; 3 mg/ml Albumin: 71,8 %
B) Direkter Einschluss von Trypan-Blau in Liposomen als Beispiel für niedermolekulare Wirkstoffe. In zwei Glasvials (Durchmesser 10 mm) wurden je 100 mg Glasperlen (Durchmesser 1 mm) sowie jeweils 50 mg der als feste Lösung vorliegenden Mischung von hydriertem Ei-Phosphatidylcholin und Cholesterol (55/45 mol%) eingewogen. In ein Vial wurde 75 µl einer Trypan-Blau Lösung (1000 µl handelsübliche Trypan Blau-Lösung (0,4 %) plus 30 ml 25%ige Ammoniaklösung) pipettiert. In das zweite Vial wurde 75 µl 0,9 % NaCl-Lösung gegeben. Die verschlossenen Vials wurden je 10 Minuten bei 3540 rpm gespeedmixt. Das mit Trypan Blau Lösung beladene Liposomen-Gel wurde mit 250 µl NaCl-Lösung redispergiert. Das nur mit Kochsalzlösung hergestellte Liposomen-Gel wurde mit 175 µl NaCl-Lösung plus 75 µl Trypan-Blau-Lösung redispergiert. Anschließend wurden beide liposomale Dispersionen in geeignete Zentrifugenröhrchen pipettert und bei 350.000 g zentrifugiert, wobei die Liposomen sedimentierten. Im Überstand wurde anschließend die Trypan-Blau-Konzentration durch Absorptionsspektroskopie bestimmt und die Einschlusseffizienz berechnet. Ergebnis Einschlusseffizienz für Trypan-Blau in Liposomen durch Speedmixen: 51 %
C) Direkter Einschluss von fluoreszenz-gelabelter RNA in neutrale Liposomen sowie in Liposomen mit negativer Oberflächenladung
   i) Neutrale Liposomen: In zwei Glasvials (Durchmesser 10 mm) wurden je 100 mg Glasperlen (Durchmesser 1 mm) sowie jeweils 50 mg der als feste Lösung vorliegenden Mischung von hydriertem Ei-Phosphatidylcholin und Cholesterol (55/45 mol%) eingewogen. In ein Vial wurde 75 µl einer 1 µM RNA-Cy3 Lösung (RNA: 27mer; 5'- GGA GCU CGC U T** C GGC GAG GUC GUG CCA-3'; T** = Thymidine-C6-Amino-NHS-Cy3; Cy3: Fluoreszenzfarbstoff; Qiagen) pipettiert. In das zweite Vial wurde 75 µl NaCl-Lösung gegeben. Die verschlossenen Vials wurden je 10 Minuten bei 3540 rpm gespeedmixt. Das mit RNA-Cy3 Lösung beladene Liposomen-Gel wurde mit 250 µl NaCl-Lösung redispergiert. Das nur mit Kochsalzlösung hergestellte Liposomen-Gel wurde mit 175 µl NaCl-Lösung plus 75 µl RNA-Lösung redispergiert. Anschließend wurden beide liposomale Dispersionen in geeignete Zentrifugenröhrchen pipettert und bei 100.000 rpm zentrifugiert, wobei die Liposomen sedimentierten. Im Überstand wurden anschließend durch Fluoreszenzspektroskopie (em: 565, ex: 515nm) die relativen Konzentrationen an fluoreszenzgelabelter RNA bestimmt und die Einschlusseffizienz für RNA-Cy3 durch Dividieren der Werte berechnet (60,1 %). Die mittlere Größe der redispergierten Vesikel wurde mit Hilfe der PCS zu 127,8 nm bestimmt.
   ii) Negativ geladende Liposomen: In zwei Glasvials (Durchmesser 10 mm) wurden je 100 mg Glasperlen (Durchmesser 1 mm) sowie jeweils 50 mg der als feste Lösung vorliegenden Mischung von hydriertem Ei-Phosphatidylcholin und Cholesterol und Dipalmitoyl-Phosphatidylglycerol (DPPG) (46/33/20 mol%) eingewogen. In ein Vial wurde 75 µl einer 1 µM RNA-Cy3 Lösung (RNA: 27mer; 5'-GGA GCU CGC U T** C GGC GAG GUC GUG CCA-3'; T** = Thymidine-C6-Amino-NHS-Cy3; Cy3: Fluoreszenzfarbstoff; Qiagen) pipettiert. In das zweite Vial wurde 75 µl NaCl-Lösung gegeben. Die verschlossenen Vials wurden je 10 Minuten bei 3540 rpm gespeedmixt. Das mit RNA-Cy3 Lösung beladene Liposomen-Gel wurde mit 250 µl NaCl-Lösung redispergiert. Das nur mit Kochsalzlösung hergestellte Liposomen-Gel wurde mit 175 µl NaCl-Lösung plus 75 µl RNA-Lösung redispergiert. Anschließend wurden beide liposomalen Dispersionen in geeignete Zentrifugenröhrchen pipettert und bei 100.000 rpm zentrifugiert, wobei die Liposomen sedimentierten. Im Überstand wurden anschließend durch Fluoreszenzspektroskopie (em: 565, ex: 515nm) die relativen Konzentrationen an fluoreszenzgelabelter RNA bestimmt und die Einschlusseffizienz für RNA-Cy3 durch Dividieren der Werte berechnet (82,9 %). Die mittlere Größe der redispergierten Vesikel wurde mit Hilfe der PCS zu 238,1 nm bestimmt.
D) Direkter Einschluss von Gemcitabine in vesikuläre Phospholipid-Gele durch Speedmixen. In eine 25 ml Injektionsflasche (Durchmesser aussen: 36 mm) wurden unter sterilen Bedingungen 1,48 g der als feste Lösung vorliegenden Mischung von hydriertem Ei-Phosphatidylcholin und Cholesterol (55/45 mol%) sowie 3 g Glasperlen (Durchmesser 1 mm) eingewogen. Die Flasche wurde zugebördelt. Durch das Septum wurden 500 µl einer Gemzar^{®}-Lösung (enthält 38,02 mg Gemcitabine/ml; Gemzar^{®} ist der Name des Gemcitabine enthaltenden Arzneimittels der Fa. Lilly, 1 Durchstechflasche enthält 200 mg Gemcitabine (lyophilisiert) und wird mit 5 ml 0,9 % NaCl rekonstituiert) sowie 2,22 g 5%ige Mannitollösung zugegeben. Die Mischung wurde 20 Minuten bei 3540 rpm gespeedmixt, wodurch ein Gemcitabine enthaltendes VPG entsteht.
   Die Einschlusseffizienz für Gemcitabine betrugt 80,7 % (Verfahren: Moog et al., Cancer Chem Pharmacol 49:356 (2002)), der Lyso-PC-Gehalt weniger als 0,5 % LysoPC/Gesamtlipid (mit Hilfe der HPTLC), die mittlere Größe der Vesikel war 38,7 nm (PCS).
   Damit konnte gezeigt werden, dass Gemcitabine durch speedmixen in VPG eingeschlossen werden kann und dass das Produkt mit Gemcitabine enthaltendem VPG vergleichbar ist, bei dem das leere VPG durch Hochdruckhomogenisation hergestellt wurde und Gemcitabine durch passives Loading (EP 1087752) nachträglich eingearbeitet wurde (Einschlusseffizienz bei passives Loading: 43-47%). Das hier durchgeführte Verfahren ist offensichtlich aber schonender, was der niedrige Lyso-PC-Gehalt der Formulierung zeigt (Gehalt bei HochdruckHomogenisation und anschließendem passivem Loading: 3-5%).
E) Direkter Einschluss von Bendamustin in vesikuläre Phospholipid-Gele durch Speedmixen. In eine 25 ml Injektionsflasche (Durchmesser aussen: 36 mm) wurden unter sterilen Bedingungen 1,0 g der als feste Lösung vorliegenden Mischung von hydriertem Ei-Phosphatidylcholin und Cholesterol (55/45 mol%) sowie 1,0 g Glasperlen (Durchmesser 1 mm) eingewogen. Die Flasche wurde zugebördelt. Durch das Septum wurde 1,5 ml einer Bendamustin^{®}hydrochloridLösung gegeben (enthielt 9,09 mg Bendamustin/ml; die Lösung wurde durch Rekonstitution von Bendamustin der Fa. Ribosepharm hergestellt. Hierzu wurde 0,9 % NaCl-Lösung in die Arzneiflasche mit dem lyophilisiertem Arzneistoff injiziert). Die Mischung wurde 30 Minuten bei 3540 rpm gespeedmixt, wobei ein Bendamustin-hydrochlorid enthaltendes VPG entstand. Es wurden Proben der Bendamustin enthaltenden VPGs entnommen und die Einschlusseffizienz für Bendamustin mit 40,6 +- 3,08 % (SD) (n: 4) bestimmt (Abtrennung des freien Bendamustins: per lonentauscher, Moog et al., Cancer Chem Pharmacol 49:356 (2002), Bendamustin-Analytik: Maas, B. et al., Pharmazie 49:775-777 (1994)). Die mittlere Größe der Vesikel wurde mit 62,5 +- 3,5 nm (SD) (n: 8) durch PCS bestimmt.
F) Direkte Herstellen eines vesikulären Phospholipid-Gels unter Verwendung einer Siliziumdioxid-Dispersion
   10 % Phospholipid in Siliziumdioxid-Dispersion: 2,25 g eines Kieselsäuregels mit 2,8 g Kieselsäureanhydrid/100 ml Wasser wurde mit 250 mg S80 versetzt. Die Mischung wurde 20 Minuten im PE-Becher (Durchmesser ca. 34 mm) bei 3540 rpm gespeedmixt. Es entstand eine cremige Formulierung, die in Wasser leicht redispergiert werden kann. Die Teilchengröße wurden mit PCS bestimmt und lag bei 31 ,5 nm.
   15 % Phospholipid in Siliziumdioxid-Dispersion: 2,15 g eines Kieselsäuregels mit 2,8 g Kieselsäureanhydrid/100 ml Wasser wurde mit 0,38 g S80 versetzt. Die Mischung wurde 20 Minuten im PE-Becher (Durchmesser ca. 34 mm) bei 3540 rpm gespeedmixt. Es entstand eine cremige Formulierung, die in Wasser leicht redispergiert werden kann. Die Teilchengröße wurde mit PCS bestimmt und lag bei 31 ,3 nm.
   30 % Phospholipid in Siliziumdioxid-Dispersion: 1,76 g eines Kieselsäuregels mit 2,8 g Kieselsäureanhydrid/100 ml Wasser wurde mit 710 mg S80 versetzt. Die Mischung wurde 20 Minuten im PE-Becher (Durchmesser ca. 34 mm) bei 3540 rpm gespeedmixt. Es entstand eine crem ige Formulierung, die in Wasser leicht redispergiert werden kann. Die Teilchengröße wurde mit PCS bestimmt und lag bei 64,5 nm.
   40 % Phospholipid in Siliziumdioxid-Dispersion: 1,49 g eines Kieselsäuregels mit 2,8 g Kieselsäureanhydrid/100 ml Wasser wurde mit 1004 mg S80 versetzt. Die Mischung wurde 20 Minuten im PE-Becher (Durchmesser ca. 34 mm) bei 3540 rpm gespeedmixt. Es entstand eine cremige Formulierung, die in Wasser leicht redispergiert werden kann. Die Teilchengröße wurde mit PCS bestimmt und lag bei 85,2 nm.

### Beispiel 6: Einarbeiten eines wasserunlöslichen Wirkstoffs in vorgefertigte VPG oder feste Lipid Nanopartikel (SLN), Screening nach einem geeigneten Wirkstoff: Lipid-Verhältnis

A) Einarbeiten in Liposomen. Es sollte untersucht werden, ob und in welchen Anteilen sich der wasserunlösliche Wirkstoff PQ013 (PQ: Prokinase GmbH, Freiburg; MG: 399,4 g/mol) in vorgefertigte Liposomen einarbeiten lässt. Hierzu wurden Liposomen-Gele aus E80 (Phosphatidylcholin-Präparation aus Ei, Fa. Lipoid, Ludwigshafen) durch Speedmixen hergestellt (500 mg E80, PE-Becher mit Durchmesser 34 mm, 750 µl 0,9 % NaCl, 10 min Speedmixen bei 3540 rpm). Steigende Mengen PQ013 wurden in Glasvials (Durchmesser 10 mm) eingewogen, bzw. bei sehr kleinen Mengen in Dioxan gelöst einpipettiert. Im letzteren Fall wurde anschließend das Lösungsmittel im Vakuum entfernt. Jeweils 125 mg des E80 Gels (Enthält 50 mg S80, ca. 68 µmol) wurden in die Vials gegeben und es wurde 30 Minuten bei 3540 rpm gespeedmixt. Anschließend wurde jeweils 1 ml PBS zugegeben und für 2 Minuten redispergiert. 100 µl der jeweiligen liposomalen Formulierungen wurden in eine 24-well Mikrotiterplatte pipettiert, kurz anzentrifugiert und anschließend mikroskopisch auf das Vorhandensein von PQ013-Kristallen untersucht. Es wurde gefunden, das bei einem molaren Verhältnis von PQ013 zu Lipid kleiner als 1:40 keine Kristalle mehr auftraten, d. h. das PQ013 in einem über 40fachen molaren Überschuss an Phospholipid (E80) vollständig löslich ist.

| Ansatz | 1 | 2 | 3 | 4 | 5 | 6 | 7 | 8 |
|---|---|---|---|---|---|---|---|---|
| PQ013 [µmol] | 68 | 6,8 | 3,4 | 1,7 | 1,13 | 0,68 | 0,14 | 0,068 |
| PQ013 [mg] | 27,2 | 2,72 | 1,36 | 0,68 | 0,45 | 0,27 | 0,06 | 0,03 |
| Verhältnis (molar) | 1:1 | 1:10 | 1:20 | 1:40 | 1:60 | 1:100 | 1:500 | 1:1000 |
| Kristalle | J | J | J | N | N | N | N | N |

Ein analoges Experiment wurde mit Liposomen aus reinem hydriertem Phosphatidylcholin aus Ei (EPC, Fa. Lipoid) durchgeführt. Dabei konnte PQ013 ebenfalls ab einem molaren Verhältnis von 30:1 Lipid zu Wirkstoff in den Liposomen gelöst werden.
B) Einarbeiten in SLN. Es sollte untersucht werden, ob und in welchen Anteilen sich der wasserunlösliche Wirkstoff PQ013 (PQ: Prokinase GmbH, Freiburg; MG: 399,4 g/mol) in vorgefertigte SLN einarbeiten lässt. Hierzu wurden SLN gemäß Beispiel 15 durch speedmixen hergestellt, allerdings wurden diese nicht redispergiert/verdünnt sondern im weiteren als viskose Masse eingesetzt. Steigende Mengen PQ013 wurden in Glasvials (Durchmesser 10 mm) eingewogen, bzw. bei sehr kleinen Mengen in Dioxan gelöst einpipettiert. Im letzteren Fall wurde anschließend das Lösungsmittel im Vakuum entfernt. Jeweils 125 mg des SLN-Gels (enthält 33,8% Trimyristin, d. h. 42,3 mg; 58,4 µmol) wurden in die Vials gegeben, die Vials wurden auf 80 °C erwärmt und es wurde 30 Minuten bei 3540 rpm gespeedmixt (6 x 5 Minuten mit zwischenzeitlicher Erwärmung). Anschließend wurde jeweils 1 ml PBS zugegeben und für 2 Minuten redispergiert. Die Proben wurden für 3 Stunden im Kühlschrank bei 4-8 °C belassen, damit die SLN fest wurden. 100 µl der jeweiligen Dispersionen wurden in eine 24-well Mikrotiterplatte pipettiert, kurz anzentrifugiert und anschließend mikroskopisch auf das Vorhandensein von PQ013-Kristallen untersucht. Es wurde gefunden, das bei einem Gewichtsverhältnis von PQ013 zu SLN kleiner als 1:500 keine Kristalle mehr auftraten. PQ013 ist in einem 500fachen molaren Überschuss an Trimyristin (SLN) vollständig löslich.

| Ansatz | 1 | 2 | 3 | 4 | 5 | 6 | 7 | 8 |
|---|---|---|---|---|---|---|---|---|
| PQ013 [µmol] | 58,4 | 5,8 | 2,92 | 1,46 | 0,98 | 0,58 | 0,12 | 0,058 |
| PQ013 [mg] | 23,4 | 2,32 | 1,17 | 0,58 | 0,39 | 0,23 | 0,05 | 0,02 |
| Verhältnis (molar) | 1:1 | 1:10 | 1 :20 | 1:40 | 1:60 | 1:100 | 1:500 | 1:1000 |
| Kristalle | J | J | J | J | J | J | N | N |

### Beispiel 7: Herstellung von Immunoliposomen durch Speedmixen

Durch dieses Beispiel soll die einfache und für das Kopplungslipid schonende Herstellung von Immunoliposomen durch Speedmixen demonstriert werden.
A) Immunoliposomen mit IgG. 37,7 mg hydriertes EPC, 10,8 mg Cholesterol, 6,6 mg DSPE-PEG-2000 und 0,44 mg DSPE-PEG-2000-Maleimid wurden in ein Glasvial (Durchmesser 10 mm) gemeinsam mit 100 mg Glasperlen (Durchmesser 1 mm) eingewogen. Die Lipide wurden mit 150 µl CHCl₃/MeOH 2:1 gelöst, anschließend das Lösemittel durch einen Stickstoffstrom wieder entfernt, so dass ein Lipidfilm zurückblieb. Lösungsmittelreste wurden über Nacht im Vakuum entfernt.
   80 µl HEPES-Puffer (20 mM HEPES, 130 mM NaCl, pH: 6,8) wurde zugegeben, das geschlossene Vial bei 37°C für 5 Minuten inkubiert und anschließend für 10 Minuten bei 3540 rpm gespeedmixt.
   Unspezifisches IgG wurde gemäß Huwyler, J. et al., Proc. Natl. Acad. Sci. USA 93:14164-14169 (1996) mit 2-Iminothiolan thioliert. Der thiolierte Antikörper wurde anschließend durch Dialyse in Borat-Puffer, pH 8,0, von überschüssigem Iminothiolan befreit und auf ein Volumen von etwa 500 µl konzentriert. Die IgG-Lösung wurde zum Liposomen-Gel gegeben und die Mischung zur Redispergierung 30 Sekunden gespeedmixt und die Mischung zwei Stunden bei RT inkubiert. Anschließend konnten die Liposomen weiter verdünnt und durch Chromatographie an Sephadex@ G50 von nicht gebundenem Antikörper getrennt werden.
B) Immunolicosomen mit einem LDL-bindenden Peptid. Es wurde zunächst ein Lipidfilm hergestellt. Hierzu wurden 120 mg EPC3/Cholesterol (55:45 mol:mol), 16,4 mg DSPE-MPEG (2000), 8 mg DSPE-PEG-Maleimid, und 500 µl einer 1mg/ml-Lösung des fluoreszierenden Farbstoffs Lissamine Rhodamine-PE in Chloroform/MeOH 2:1 (d.h. 0,5 mg Lissamine) in einem 25 ml Kolben gegeben und mit ca. 5 ml CHCl₃/MeOH 2:1 in Lösung gebracht. Das Lösungsmittel wurde mit Hilfe eines Rotationsverdampfers entfernt, so dass ein rötlich-glasiger Lipidfilm zurückblieb. Dieser wurde über Nacht im Vakuum getrocknet, aus dem Kolben gekratzt und unter trockenen Bedingungen aufbewahrt.
   20 mg dieser Lipidmischung wurden in ein 10 ml Glasvial zum Zubördeln (Durchmesser aussen 20 mm) gegeben, in dem sich 100 mg Glasperlen (Durchmesser 1 mm) befanden. Durch das Septum wurden 40 µl einer 0,35 M Fosphenytoin-Lösung (in 150 mM Borat-Puffer plus 100 mM EDTA, pH 8) gegeben und das Gemisch wurde zunächst 30 s, nach weiteren 5 min dann für 5 min bei 3540 rpm gespeedmixt. Anschließend wurde 50 µl einer Peptidlösung, enthaltend 30 nmol eines 26 AS-Peptids aus der Bindungsregion des humanen ApoA4 mit einer Mercaptopropionsäure am N-Terminus (zur Bindung an die Maleimidgruppe auf den Liposomen) sowie 100 µl 150 mM Boratpuffer (plus 100 mM EDTA) zu der viskosen Liposomendispersion gegeben und 2 min gespeedmixt. Es wurde über Nacht bei RT inkubiert, die Liposomendispersion anschließend mit 2 ml PBS (1 mM, pH 6 plus 150 mM NaCl) verdünnt und das ungebundene Peptid und das nicht eingeschlossene Fosphenytoin durch Gelfiltration an Sephadex@ G-59 Fine abgetrennt.

### Beispiel 8: Gleichmäßiges und rasches Einarbeiten von Wirkstoffen zwischen die Vesikel von vorgefertigten VPG durch Speedmixen

Bei diesen Experimenten sollte gezeigt werden, dass Wirkstoffe/Farbstoffe rasch in bereits vorgefertigte VPG eingearbeitet werden können (VPG-Herstellung z. B. durch Hochdruckhomogenisation). Ziel ist die gleichmäßige Verteilung des Wirk-/ Farbstoffs zwischen den Vesikeln, was eine wichtige Voraussetzung für das passive loading darstellt. In den folgenden Beispielen wurde das Einarbeiten von Trypan-Blau als Modellwirkstoff sowie von Gemcitabine als relevanter Wirkstoff zwischen die Vesikel bei vorgefertigten VPGs durch Speedmixen untersucht.
A) Rasches Einarbeiten eines Modellwirkstoffs (Trypan-Blau). In eine 25 ml Injektionsflasche aus Klarglas wurde 1 g VPG (660 mM Lipid, Mischung von hydriertem Ei-Phosphatidylcholin und Cholesterol (55/45 mol%), als feste Lösung vorliegend) eingefüllt, und die Flasche kurz zentrifugiert, so dass sich eine gleichmäßig dicke Schicht des VPG am Gefäßboden ausbildete. Zu dieser Schicht wurden 135 µl einer 0,04%igen Trypan-Blau-Lösung gegeben und das Gefäß für 1 Minute bei 3540 rpm gespeedmixt. Durch genaue Sichtprüfung wurde festgestellt, das der Farbstoff gleichmäßig in das VPG eingearbeitet wurde.
B) Rasches Einarbeiten von Gemcitabine. In eine 25 ml Injektionsflasche wurde 3,7 g VPG (660 mM Lipid, Mischung von hydriertem Ei-Phosphatidylcholin und Cholesteroi (55/45 mol%), als feste Lösung vorliegend) eingefüllt, und die Flasche kurz zentrifugiert, so dass sich eine gleichmäßig dicke Schicht des VPG am Gefäßboden ausbildete. Zu dieser Schicht wurden 500 µl µl einer Gemzar^{®}-Lösung gegeben (enthält 38,02 mg Gemcitabine/ml; Gemzar® ist der Name des Gemcitabine enthaltenden Arzneimittels der Fa. Lilly, 1 Durchstechflasche enthält 200 mg Gemcitabine (lyophilisiert) und wird mit 5,0 ml 0,9 % NaCl rekonstituiert). Das Gemisch wurde bei 3540 rpm 0,5 Minuten gespeedmixt.
   Anschließend wurde das VPG-Gemcitabine-Gemisch sowie eine Vergleichsprobe, bei der Gemcitabine durch zweimaliges Schütteln über 10 und 5 Minuten und dazwischenliegender 1-stündiger Inkubationpause bei Raumtemperatur in das VPG eingearbeitet wurde, zum passiven Loading 2 Stunden bei 60 °C inkubiert, anschließend das VPG redispergiert und die Einschlusseffizienz für Gemcitabine bestimmt.
   Einschlusseffizienz bei Einarbeiten des Gemcitabine durch Speedmixen: 44 %.
   Einschlusseffizienz bei Einarbeiten des Gemcitabine durch Schütteln: 47 %.

### Beispiel 9: Schnelles und sicheres Redispergieren von leeren und wirkstoffhaltigen viskosen, partikelenthaltenden Wirkstoffformulierungen durch Speedmixen/Mischen mittels DAZ

A) Redispergieren eines VPG durch Speedmixen bei mehrstufiger Zugabe des Verdünnungsmediums (Redispergiermediums)
   (i) 3,7 g eines VPG (hergestellt durch Hochdruckhomogenisation (700 bar, 10 Zyklen) einer als feste Lösung vorliegenden Mischung von hydriertem Ei-Phosphatidylcholin und Cholesterol (55/45 mol%)) wurden in eine 25 ml Injektionsflasche eingewogen und kurz zentrifugiert. Anschließend wurde für das Redispergieren zunächst 500 µl NaCl-Lösung zugegeben und für 1 Minute gespeedmixt. Anschließend erfolgte die Zugabe von 6,4 ml NaCl-Lösung und es wurde wieder 1 Minute gespeedmixt. Die entstandene Dispersion war homogen und frei von großen Partikeln, die Vesikelgrößenbestimmung durch PCS ergab 28,1 nm.
   (ii) Zu 4,2 g eines Gemcitabine enthaltenden VPGs (hergestellt durch Hochdruckhomogenisation (700 bar, 10 Zyklen) einer als feste Lösung vorliegenden Mischung von hydriertem Ei-Phosphatidylcholin und Cholesterol (55/45 mol%) und anschließendes passives Loading mit Gemcytabine-Hydrochlorid) wurden in einer 25 ml Injektionsflasche zunächst 500 µl NaCl-Lösung zugegeben und für 1 Minute gespeedmixt. Anschließend erfolgte die Zugabe von 5,9 ml NaCl-Lösung und es wurde wieder 1 Minute gespeedmixt. Die entstandene Dispersion war homogen und frei von großen Partikeln, die Vesikelgrößenbestimmung durch PCS ergab 37,8 nm.
B) Redispergieren eines VPG durch einmalige Zugabe des Verdünnungsmediums. 1,0 g eines VPG (hergestellt durch Hochdruck-homogenistaion einer als feste Lösung vorliegenden Mischung von hydriertem Ei-Phosphatidylcholin und Cholesterol (55/45 mol%)) wurde in ein 25 ml Injektionsflasche eingewogen, es wurden 2 g Glasperlen (Durchmesser ca. 5 mm) zugegeben und kurz zentrifugiert. Anschließend wurde für das Redispergieren 2,0 ml NaCl-Lösung zugegeben und für 2 Minuten gespeedmixt. Die entstandene Dispersion war homogen und frei von großen Partikeln, die Vesikelgrößenbestimmung durch PCS ergab 35,9 nm.
C) Redispergieren durch Zugabe des Verdünnungsmediums mit Hilfe eines Applikators. Hier wurde das Redispergiermedium durch einen Applikator (Fig. 4) zugegeben: In ein Glasvial (Innendurchmesser ca. 13 mm) wurde ein 0,5 ml Eppendorf-Gefäß gesteckt, das am unteren Ende eine kleine Bohrung aufwies. Die Bohrung war so klein, das keine wässerige Lösung durchtropfen konnte. In das Eppendorf-Gefäß wurde 250 µl der 5% igen Mannitollösung gegeben (Redispergiermedium) und in das Glasvial 125 mg eines VPG (hergestellt durch Hochdruck-homogenistaion einer als feste Lösung vorliegenden Mischung von hydriertem Ei-Phosphatidylcholin und Cholesterol (55/45 mol%)). Das Glasvial wurde verschlossen, wodurch gleichzeitig das Eppendorf-Gefäß fixiert wurde. Das Konstrukt wurde für 30 Sekunden gespeedmixt. Die entstandene Dispersion war homogen und frei von großen Partikeln, die Vesikelgrößenbestimmung durch PCS ergab 36,8 nm.
D) Redispergieren einer Liposomen-enthaltenden Creme. 200 mg "Aloe Vera Liposomen Gel" der Fa. Anton Hübner wurde in einen PE-Becher (Innendurchmesser ca. 14 mm) eingewogen und mit 2 ml Wasser versetzt. Die Mischung wurde für 2 Minuten gespeedmixt, die entstandene Dispersion war homogen und frei von großen Partikeln.
E) Probenvorbereitung bei der Größenbestimmung bei einer Siliziumdioxid-Dispersion Es wurde 1 g einer SiO₂-Dispersion (2,8 g SiO₂/100ml) mit 1 g Glasperlen (Durchmesser 1 mm) sowie 9 ml Wasser versetzt und im PE-Becher (Innendurchmesser ca. 34 mm) für 10 Minuten gespeedmixt. Anschließend konnte eine Größenbestimmung mit PCS durchgeführt werden und ergab eine gut meßbare Partikelgröße von 55 nm.

### Beispiel 10: Reproduzierbares Herstellen einer Surfactantdispersion mit kleinen Vesikeln aus lyophilisierten Surfactant durch Speedmixen

Zwei Fläschchen Alveofact Trockenampulle wurden für das Experiment verwendet (Innendurchmesser ca. 13 mm). Ein Fläschchen wurde gemäß der Herstellervorschrift mit der vorgeschriebenen Menge NaCl-Lösung rehydratisiert und extrudiert (siehe Alveofact Trockenampulle Fachinformation). Ein zweites Fläschchen wurde mit der gleichen Menge NaCl-Lösung versetzt, danach aber 5 Minuten im Speedmixer® bei 3540 rpm behandelt. Die mittlere Größen der entstandenen Vesikel wurden durch PCS bestimmt und verglichen. Zudem wurden die Dispersionen 9 Tage bei 4 °C aufbewahrt und dann nochmals vermessen.
Vial I (Herstellung nach Herstellerangabe):
   - starke Schaumbildung, genaues Abmessen der Suspension nicht möglich.
   - Partikel: 67,5 nm (die PCS-Messung ergibt eine sehr breite Verteilung, die bis 5 µm reicht). Vereinzelt noch kleine Flocken zu sehen.
   - Nach 9 Tagen bei 4-8 °C: Es hat sich ein Niederschlag gebildet. Partikelgrößen wurden mit 132,9 nm bestimmt, was ein deutliches Partikelwachstum bedeutet. Auch die enorme Breite der Verteilung (sehr viele Partikel haben Partikeldurchmesser von über 5 µm) lässt auf das Vorhandensein von sehr großen Partikeln schließen.
Vial II (Herstellung durch Speedmixen):
   - kein Schaum, sauberes Abmessen möglich.
   - Partikel: 48,1 nm (deutlich schmalere Partikelverteilung, die im PCS bis 1 µm reicht)
   - Dispersion scheint den Kontakt zur Glaswand zu scheuen, Bildung von großen Vakuolen beim Schütteln: Dies bedeutet eine sehr verringerte Oberflächenspannung der Dispersion, entsprechend dem Lungen-Surfactant.
   - Nach 9 Tagen bei 4-8°C: Partikelgröße: 48,8 nm. Damit hat sich die primär hergestellte Dispersion nicht geändert. Es wurde im Gegensatz zu Vial I kein Niederschlag beobachtet.

### Beispiel 11 : Herstellen von Liposomen aus Lösungen von Lipiden in organischen Lösungsmitteln und einer wässerigen Phase durch Speedmixen

Bei diesem Beispiel wurden Liposomen durch eine Kombination der Injektionsmethode mit der Liposomenherstellungsmethode durch Speedmixen kombiniert. Zu diesem Zweck wurde ein Glasvial (Innendurchmesser ca. 13 mm) mit 100 mg Glasperlen (Durchmesser ca. 1 mm) gefüllt. In den Hals des Vials wurde ein 0,5 ml Eppendorf-Gefäß gesteckt, das am unteren Ende eine kleine Bohrung aufwies. Die Bohrung war so klein, das weder Wasser noch Ethanol durchtropfte.
A) Vorlegen der Lipide in organischen Lösungsmitteln, Zugabe der wässerigen Phase durch Applikator. In das Glasvial wurde eine Lösung von 80 mg S80 in 100 µl Ethanol eingefüllt. Das Eppendorf-Gefäß wurde aufgesteckt und mit 400µl NaCl-Lösung befüllt. Das Konstrukt wurde zugeschraubt, wodurch auch das aufgesteckte Eppendorf-Gefäß stabilisiert wurde. Anschließend wurde bei 3540 rpm für 5 Minuten gespeedmixt, wobei ein leicht trübes, stark viskoses Gel entstand. In das obere Eppendorf-Gefäß wurden weitere 500 µl NaCl-Lösung gegeben und das Konstrukt weitere 5 Minuten gespeedmixt. Es entstand eine liposomale Formulierung, die mittlere Größe der Liposomen wurde mit Hilfe der PCS zu 122,7 nm bestimmt.
B) Vorlegen der Lipide in organische Lösungsmitteln, schrittweise Zugabe der wässerigen Phase. Versuch I wurde in dem Sinne wiederholt, als das zur ethanolischen Lösung von S80 die ersten 400 µl NaCl-Lösung in 100 µl Schritten zugegeben wurden und die Mischung jeweils für 2 Minuten gespeedmixt wurde. Anschließend wurden, wie bei Versuch I, die restlichen 500 µl NaCl-Lösung auf einmal zugegeben und nochmals 5 Minuten gespeedmixt. Die mittlere Größe der entstandenen Liposomen wurde mit 116,1 nm bestimmt.
C) Vorlegen der wässerigen Phase, Zugabe der Lipide in organischen Lösungsmitteln mit Applikator. In das Glasvial wurden 900 µl NaCl-Lösung eingefüllt, in das aufgesteckte Eppendorf-Gefäß eine Lösung aus 80 mg S80 in 100 µl Ethanol. Das Konstrukt wurde für 10 Minuten gespeedmixt. Die enstandenen Liposomen wurden mit Hilfe der PCS mit 83,9 nm bestimmt.

### Beispiel 12: Sicheres und rasches Einarbeiten von Liposomen in Salben, Cremes und Gele und deren Grundlagen

In eine handelsübliche Creme (Tagescreme mit Nachtkerzenöl der Fa. Anton Hübner) wurden Siliziumdioxid-Partikel enthaltende Dispersionen eingearbeitet. Zur Verwendung kamen Dispersionen, die ca. 2 g Siliziumdioxid/100 ml enthielten sowie 10, 15, 30 und 40 % des Phosholipids S 80 (Herstellung siehe Bsp. 5F). Hierzu wurden 1,2 - 2,3 g der Creme in einen PE-Becher (Durchmesser innen ca. 34 mm) eingewogen und mit verschiedenen Anteilen von 10 - 50 Gew% der Siliziumdioxid-Dispersionen versetzt und anschließend 20 Minuten bei 3540 rpm gespeedmixt. In allen Versuchen wurden homogene Cremes erhalten.

| Creme [g] | Siliziumdioxid-S80-Dispersion (Gewicht [g] und Gewichts-%) | S80-Anteil in der Silizumdioxid-S80-Dispersion [%] |
|---|---|---|
| 1,19 | 1,22 (50) | 10 |
| 1 ,40 | 0,27 (20) | 10 |
| 2,26 | 0,24 (10) | 40 |
| 2,24 | 0,23 (10) | 30 |
| 2,27 | 0,22(10) | 15 |

### Beispiel 13: Erleichterung der Hochdruckhomogenisation durch DAZ-Vorbehandlung

7,0 g einer als feste Lösung vorliegenden Mischung von hydriertem Ei-Phosphatidylcholin und Cholesterol (55/45 mol%) wurde in eine PE-Becher (Innendurchmesser ca. 51 mm) eingewogen und mit 14 ml 5%iger Mannitollösung versetzt. Die Mischung wurde 20 Minuten bei 3540 rpm gespeedmixt, anschließend wurde die dann homogene Mischung der Hochdruckhomogenisation unterworfen (3 Zyklen). Das resultierende VPG wurde redispergiert und die Vesikelgrößen mit 28,9 nm bestimmt (PCS), was der üblichen Partikelgröße in einem VPG entspricht, das nur durch Hochdruckhomogenisation hergestellt wurde (10 Zyklen).

### Beispiel 14: Herstellung von Emulsionen durch Speedmixen

Es wurden Emulsionen aus Sojaöl (10%) in Wasser mit Phospholipid S75/S80 als Em ulgator (2,4% und 1,2%) hergestellt.
A) Emulsion mit 2,4 % Emulgator. 2 ml einer Dispersion von S75 (2,66 g S75/l) werden mit 220 µl Sojaöl in einem PE-Becher (Innendurchmesser ca. 14 mm) zusammengeführt und bei Raumtemperatur 5, 10 und 15 Minuten gespeedmixt (3540 rpm). Die Emulsionen wurden mit der PCS vermessen. Nach einer Mix-Zeit von 10 Minuten wurde auch die Teilchenzahl > 1 µm mit Hilfe des Abschattungsverfahrens bestimmt. Zu Vergleichszwecken wurde auch die Lipiddispersion der Lipidemuision NuTRI flex^{®} der Fa. B. Braun verm essen.
Bereits nach 5 Minuten hatte sich eine Emulsion gebildet mit einer Teilchengröße um 50 nm, die sich auch durch weiteres Speedmixen nicht änderte. Die Anzahl der Teilchen > 1 µm der so hergestellten Emulsion betrug bei einer Verdünnung von 1:20 Mill. 48 (pro ml). Das NuTRIflex^{®}-Produkt hatte ähnlich viele Teilchen in diesem Größenbereich (58, gleiche Verdünnung).

| Exp. | Mix-Zeit | Teilchengröße | Partikel pro ml (Verdünnung 1:20 Mill.) |
|---|---|---|---|
| 22a | 5 | 53,8 | 53 |
| 22b | 10 | 55,4 | 48 |
| 22c | 15 | 51,8 | 66 |
| NuTRIflex^{®} | - | 197,0 | 58 |

B) Emulsion mit 1,2 % Emulgator. 1 g Sojaöl und 120 mg S80 wurden in einem PE-Becher (Innendurchmesser ca. 14 mm) zusammengegeben und 1,8 g Glasperlen (Durchmesser 1mm) und 1 ml Wasser zugefügt. Die Mischung wurde auf 60 °C erwärmt und anschließend 5 Minuten gespeedmixt (3540 rpm). Durch Zugabe von 9 ml Wasser wurde die Emulsion auf 10 % eingestellt. Die Partikelgröße wurde mit Hilfe von PCS zu 49,2 nm bestim mt.

### Beispiel 15: Herstellung von festen Lipid Nanohartikeln (SLN) durch Speedmixen

Es wurden SLN aus Trimyristin (10%) in Wasser und Tyloxapol als Emulgator (6 %) hergestellt. 1 g Trimyristin und 560 mg Tyloxapol wurden mit 1.400 µl Wasser in einem PE-Becher (Innendruchmesser ca. 34 mm) zusammengegeben. Es wurden 1,5 g Glasperlen (Durchmesser 1 mm) zugegeben und das Gemisch auf 80°C erwärmt. Anschließend wurde 5 Minuten bei 3540 rpm gespeedmixt. Die Erwärmung des viskosen Gemisches und das Speedmixen wurde noch dreimal wiederholt. Anschließend wurde durch Wasserzugabe die Dispersion auf 10 % Trimyristin eingestellt und für 12 Stunden bei 4-6°C im Kühlschrank belassen. Die Teilchengröße wurde mit Hilfe der PCS mit 69,8 nm bestimmt. Die Anzahl der Teilchen > 1 µm der so hergestellten SLN betrug bei einer Verdünnung von 1:20 Mio lediglich 9 Partikel. Ein für die klinische Routine zugelassene Lipiddispersion (NuTRIflex^{®} von B. Braun, siehe Beispiel 1.12) hatte eine weit höhere Anzahl Teilchen in diesem Größenbereich (58, gleiche Verdünnung).

### Beispiel 16: Redispergieren mittels DAZ-Mischvorrichtung

125 mg Liposomen-Gel, hergestellt durch 20 Minuten Speedmixen einer Mischung von 50 mg hydriertem Ei-Phosphatidylcholin und Cholesterol (55/45 mol%) und 75 µl 5%iger Mannitollösung sowie 100 mg Glasperlen, wurde mit 250 µl Mannitollösung versetzt, in einem Eppi in den Mischbehälter wie in Fig. 3 in den Speedmixer gegeben und für 20 Sekunden gespeedmixt. Die redispergierte Liposomendispersion war homogen und frei von großen Partikeln, die durch PCS bestimmte mittlere Größe der Vesikel betrug 74,2 nm.

### Beispiel 17: Gewebeaufschluss mittels DAZ

Das in Beispiel 16 beschriebene Verfahren kann auch zur Zertrümmerung von biologischen Proben (z.B. Gewebe) eingesetzt werden. Insbesondere können mit dieser Technik auch sehr kleine Probenmengen zertrümmert werden. Hierzu wurden tieftemperatur-geeignete Aufnahmebehälter für Probenvials hergestellt (siehe Fig. 10 bis 12), in die verschiedene Typen von sog. Kryo-Vials und Eppis fest eingespannt werden können. Die folgenden Experimente wurden mit 2 ml Kryovials von Greiner durchgeführt. Hierzu wurden kleine Stücke Gewebe (Muskelfleisch vom Rind) in die Kryovials gegeben, es wurden Glasperlen zugefügt, die Vials verschlossen und in flüssigem Stickstoff eingefroren (Art/Menge der Glasperlen und Gewicht der Gewebe siehe Tab. 5).

Anschließend wurden die Vials in den vorgekühlten Aufnahmebehälter gedrückt, dieser in den Speedmixer eingesetzt und für 30 s bei 3450 rpm gespeedmixt. Dann wurden die Vials entnommen, ihr Inhalt geprüft und anschließend erneut für 30 s gespeedmixt. Falls notwendig wurde zwischendurch erneut abgekühlt. Dies wurde so lange wiederholt, bis der Inhalt der Vials ein feines Pulver war bzw. bis sich der Inhalt der Vials von Intervall zu Intervall nicht mehr sichtbar veränderte. Die Ergebnisse sind in Tab. 5 zusammengefasst. Das rötlich-weiße Gewebepulver kann das zur weiteren Verarbeitung (z.B. Lipidextraktion oder RNA-Extraktion) eingesetzt.

**Tabelle 5: Gewebeaufschluss mit Speedmixer**

| Experiment | Gewicht Gewebe | Glasperlen (Durchmesser/Menge) | Ergebnis |
|---|---|---|---|
| 1 | 82 mg | 1 mm, 103 mg | Nicht einfach zertrümmerbar, große Brocken, Oberfläche pulvrig |
| 2 | 55 mg | 3 mm, 136 mg/3 Stück | 60 Sekunden - feines Pulver |
| 3 | 55 mg | 5 mm, 171 mg/1 Stück | 60 Sekunden - feines Pulver |
| 4 | 208 mg | 1 mm, 96 mg | Nicht einfach zertrümmerbar, große Brocken, Oberfläche pulvrig |
| 5 | 216 mg | 3 mm, 142 mg/3 Stück | 120 Sekunden, feines Pulver mit kleinen Krümeln |
| 6 | 243 mg | 5 mm, 196 mg/1 Stück | 90 Sekunden - feines Pulver |
| 7 | 69 mg | Mischung 5 mm (1) und 1 mm (52 mg) | 60 Sekunden - feines Pulver |
| 8 | 232 mg | Mischung 5 mm (1) und 1 mm (61 mg) | 60 Sekunden - feines Pulver |

Die Ergebnisse zeigen, das insbesondere kleine Mengen in dem hier genutzten Vial rasch und vollständig zertrümmert werden können. Wichtig ist aber auch die Nutzung der richtigen Zertrümmerungs-Hilfe. In diesem Experiment haben sich die größten Glasperlen (hier 5 mm) als am günstigsten erwiesen. Offensichtlich haben sie aufgrund ihres Gewichtes den größten Effekt, denn das Verfahren basiert auf der Beschleunigung der Zertrümmerungshilfe. Ebenfalls als günstig hat sich die Kombination von kleinen und großen Glasperlen erwiesen (siehe Tab. 5, Nr. 7 und 8). Hier konnten bei beiden Probengrößen (< 100 mg / > 200 mg) gute Ergebnisse erzielt werden.

Um die Nutzbarkeit dieser Technik im Bereich der Lebensmittelanalytik/-kontrolle aufzuzeigen, wurde ein Käse (Parmigiano Reggiano der Fa. Gucina, 32 % Fett i.Tr. (Aldi-Süd)) durch die oben beschriebene Technik in ein feines, gelblich-weißes Pulver überführt. Hierzu waren insgesamt 60 s Speedmixen bei 3450 rpm notwendig. Es wurden zwei Ansätze durchgeführt (86 und 81 mg Parmesan-Käse), wobei zum einen Ansatz drei 3-mm Glaskugeln, zum anderen eine 5 mm Glaskugel gegeben wurden. Die Ergebnisse waren nicht unterscheidbar.

Um die Nutzbarkeit dieser Technik im Bereich der (pharmazeutischen) Analytik aufzuzeigen, wurde zur Probenvorbeitung eine halbe Tablette Paracetamolratiopharm@ 500 bei Raumtemperatur in ein Eppendorf-Gefäß gegeben. Als Zertrümmerungs-Hilfe dienten zwei Stahlkugel, Durchmesser 3 mm. Durch die oben beschriebene Technik wurde innerhalb von 30 s ein feines weißes Pulver erzeugt, das sich jetzt einfach für die weitere Analytik einwiegen ließ. Die Technik kann also als Ersatz für einen Mörser genutzt werden.

### Beispiel 18: Herstellung von Liposomen in Injektionsflaschen

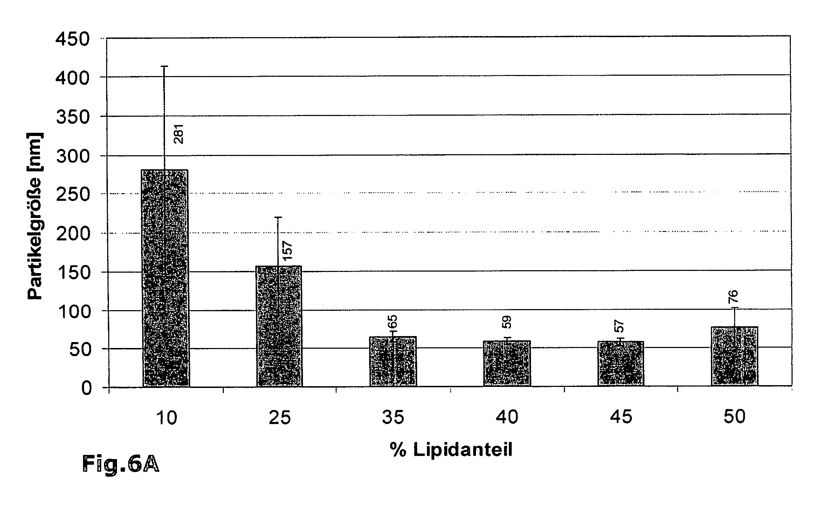

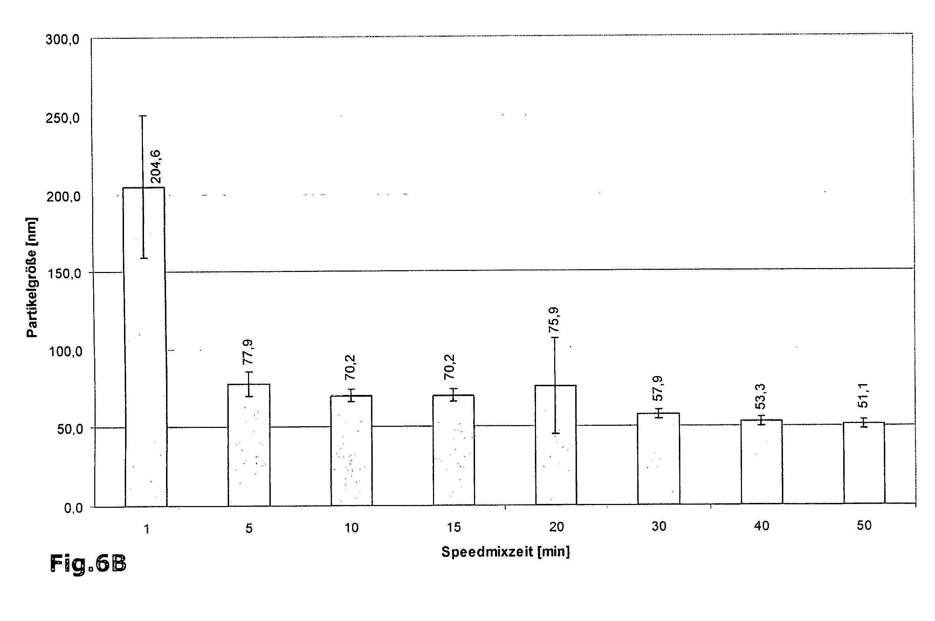

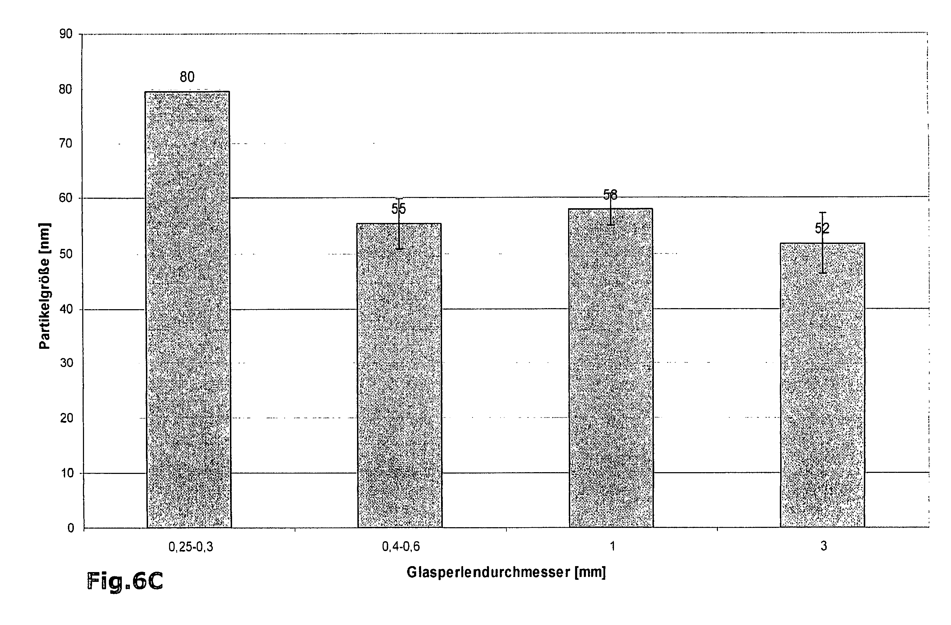

Bei zukünftigen "Bedside-Preparations" von liposomalen Formulierungen werden aus heutiger Sicht Injektionsfläschchen aus Glas zum Einsatz kommen. Aus diesem Grunde wurden auf Basis solcher Gefäße (25 m l Injektionsflasche, Aussendurchmesser 36 mm) Experimente (i) zum Einfluß der Lipidkonzentration während des Speedmixens, (ii) zum Einfiußder Speedmix-Zeit und (iii) zum Einfluß unterschiedlicher Homogenisierungs-Hilfen (Glaskugeln mit unterschiedlichem Durchmesser) auf den Homogenisiererfolg (Vesikelgröße) durchgeführt.
(i) Einfluß der Lipidkonzentration: Jeder Ansatz enthielt 0,5 g einer als feste Lösung vorliegenden Mischung von hydriertem Ei-Phosphatidylcholin und Cholesterol (55/45 mol%) sowie 0,5 g Glasperlen (Durchmesser 1 mm) als Homogenisier-Hilfe. Es wurde soviel 0,9%ige Kochsalzlösung zugegeben, dass Dispersionen mit 10, 25, 35, 40, 45 und 50 Gew.% an Lipid enstanden. Diese Dispersionen wurden jeweils 20 min bei 3540 rpm gespeedmixt, anschließend wurden die Vesikelgrößen mit Hilfe der PCS bestimmt. Jedes Experiment wurde viermal durchgeführt. Die Ergebnisse sind in Fig. 6A dargestellt. Es zeigte sich, dass die kleinsten Vesikel mit Lipidkonzentrationen von 35-45 % herstellbar sind (57-65 nm). In diesem Konzentrationsbereich sind die Ergebnisse auch am reproduzierbarsten, was an den kleinen Standardabweichungen zu erkennen ist.
(ii) Einfluß der Speedmix-Zeit: Es wurde wie unter (i) beschrieben gearbeitet, allerdings wurde in allen Experimenten mit dem gleichen Lipidkonzentration von 40%. Die Speedmix-Zeiten waren 1, 5, 10, 15, 20, 30, 40 und 50 min. Es konnte eine Verkleinerung der Vesikelgrößen mit steigender Speedmixdauer gezeigt werden (Fig. 6B). Nur nach 1 min herrschten sehr große und heterogen größenverteilte Vesikel vor. Bereits nach 5 Minuten waren Vesikel mit einer Größe kleiner als 80 nm nachweisbar. Nach 50 Minuten hatten die Vesikel eine Größe, die knapp über 50 nm lag und sich nicht stark von den Größen bei 30 und 40 Minuten Speedmixdauer unterschieden.
(iii) Einfluß der Dispergierhilfen: Es wurde wie unter (ii) beschrieben gearbeitet, allerdings in allen Experimenten mit der gleichen Lipidkonzentration (40%) und der gleichen Speedmix-Zeit (30 min). Variiert wurde die Art der Glasperlen, die als Aufschüttelhilfe zu den Lipidmischungen gegeben wurden. Es wurde jeweils 0,5 g Glasperlen mit den Durchmessern 0,25-3 mm, 0,4-0,6 mm, 1 mm oder 3 mm zugegeben. Es konnte gezeigt werden, das die Glasperlen mit dem kleinsten Durchmesser zum schlechtesten Homogenisiererfolg führten, die Vesikel hatten eine Größe von etwa 80 nm (Fig. 6C). Glasperlen von 0,4 mm bis 3 mm zeigten keinen signifikant unterschiedlichen Effekt auf das Homogenisierergebnis, die Vesikelgrößen lagen zwischen 52 und 58 mm.

### Beispiel 19: Abhängigkeit der erzielbaren Vesikelgrößen von der DAZ-Geschwindigkeit

Um die Abhängigkeit der Teilchengrößen von der Geschwindigkeit des Speedmixers und damit von der Intensität des Homogenisiervorganges zu zeigen, wurden wie in Beispiel 18 (Liposomen-Herstellung in injektionsfläschchen) beschrieben Liposomen hergestellt. Dabei wurde immer 20 min gespeedmixt, die Speedmixgeschwindigkeit wurde bei den Experimenten von 2000 rpm bis zu 3450 rpm - der maximalen Geschwindigkeit des zur Verfügung stehenden Speedmixers - variiert. Das Rückdrehverhältnis lag konstant bei 4,2:1. Die Ergebnisse dieser Experimente sind in Fig. 7 dargestellt.

Die Untersuchung zeigt, dass die erzielbaren Vesikelgrößen von der Speedmixgeschwindigkeit abhängen und dass durch Steigerung der Speedmixgeschwindigkeit kleinere Vesikelgrößen erzielbar sind. Relevante Geschwindigkeiten für die Herstellung insbesondere von kleinen Vesikeln unter 100 nm liegen im Bereich über 2000 rpm. Durch Berechnung einer Ausgleichsfunktion kann zudem gezeigt werden, dass bei weiterer Steigerung der Geschwindigkeit Teilchengrößen im Bereich von 30 nm realisierbar sein sollten (zwischen 4000 und 5000 rpm).

### Beispiel 20: Arbeiten mit geringen Ansatzgrößen

Ein wichtiger Aspekt der Herstellung von lipidbasierten Nanopartikeln wie z.B. Liposomen ist, das man mit der DAZ-Technik unter sterilen Bedingungen auch sehr kleine Mengen, wie sie in Tierversuchen oder im Bereich der Zellkultur benötigt werden, verarbeiten kann. Um dies zu zeigen, wurde ein Experiment durchgeführt, bei dem in einem 10 ml Probenglas mit Rollrand (Typ N20-10 DIN (10 ml), Macherey-Nagel), das unter sterilen Bedingungen gefüllt und zugebördelt werden kann, Liposomen aus einer EPC-3/Cholesterol-Mischung (siehe Beispiel 1) hergestellt wurden. Als Ansatzgrößen wurden 40, 20, 10 und 5 mg Lipid gewählt. In jedes Vial wurde zudem 120 mg Glasperlen (Durchmesser 1 mm) als Homogenisierhilfe gegeben. Es wurde jedem Vial 0,9%ige Kochsalzlösung zugesetzt, das Volumen entsprach dabei 150 % des Gewichtes des eingewogenen Lipids. Die Lipidgewichte und die zugegebene Flüssigkeitsmenge sind in Tab. 6 dargestellt. Die Proben wurden jeweils 30 s gespeedmixt, dann für 25 min bei Raumtemperatur belassen. Anschließend wurden die Proben nochmals 5 min bei 3450 rpm gespeedmixt. Es wurde mit Kochsalzlösung auf einen Lipidgehalt von 10 mM verdünnt (Zugabe von 6,86 ml, 3,43 ml, 1,80 ml bzw. 0,95 ml Kochsalzlösung, anschließend 3 min speedmixen), anschließend wurden die Vesikelgrößen mit Hilfe der PCS bestimmt (Tab. 6). Alle Vesikelgrößen, auch die bei den sehr kleinen Ansätzen, waren untereinander vergleichbar und zudem sehr klein.

**Tabelle 6: Vesikelgrößen bei kleinen Ansatzgrößen**

| Experiment | Lipidmenge | Volumen 0,9%ige Kochsalzlösung | Vesikelgröße |
|---|---|---|---|
| 1 | 39,9 mg | 59,9 µl | 51,2 nm |
| 2 | 10,02 mg | 30,0 µl | 49,8 nm |
| 3 | 10,48 mg | 15,7 µl | 44,2 nm |
| 4 | 5,53 mg | 8,3 µl | 51,5 nm |

## Patentansprüche

1. Verwendung einer Mischvorrichtung für chemische und/oder biologische Substanzen mit
einer ersten Antriebseinrichtung (12) zum Drehen eines Auslegers (16) um eine erste Drehachse (18),
einem in einem Abstand zur ersten Drehachse (18) mit dem Ausleger (16) verbundenen Mischbehälter (40) zur Aufnahme der Substanzen und
einer zweiten Antriebseinrichtung (30) zum Drehen des Mischbehälters (40) um eine zweite, durch den Mischbehälter (40) verlaufende Drehachse (28) wobei die Innenwände (46) des Mischbehälters (40) unterschiedliche Abstände zur zweiten Drehachse (28) aufweisen, und/oder
wobei eine innerhalb des Aufnahmebehälters (24) angeordnete Halteeinrichtung (72, 80) zum lagegenauen Halten des Mischbehälters (40) in dem Aufnahmebehälter (24) dient, und/oder
eines Mischbehälters für eine dualen asymmetrischen Zentrifuge (DAZ) mit einem ersten Aufnahmeraum (50) zur Aufnahme einer ersten Substanz und mindestens einem zweiten Aufnahmeraum (52) zur Aufnahme einer zweiten Substanz, wobei in einer Trennwand (70) zwischen den Aufnahmeräumen (50, 52) eine Öffnung (68) vorgesehen ist, um beim Auftreten von Zentrifugalkräften ein Überführen einer der Substanzen in den anderen Aufnahmeraum (50, 52) zu ermöglichen, zur Herstellung von lipidbasierte Nonopartikeln,
zum Durchmischen von ein- oder mehrphasigen biologischen, substanzen zum Zellaufschluss und zur Aufarbeitung von Geweben in einer dualen asymaretrischen zentrifuge (DAZ).

2. Verwendung nach Anspruch 1, wobei in der Mischvorrichtung der Mischbehälter (40) im Wesentlichen zylindrisch ausgebildet ist, und wobei vorzugsweise eine Längsachse (47) des Mischbehälters (40) in einem Winkel zur zweiten Drehachse (28) angeordnet ist, und wobei der Winkel vorzugsweise im Bereich von 70 bis 110° liegt.

3. Verwendung nach Anspruch 1 oder 2, wobei die Mischvorrichtung einen Aufnahmebehälter (24) zur Aufnahme des Mischbehälters (40) aufweist, wobei der Mischbehälter (40) vorzugsweise zylindrisch ist und die Längsachse des Aufnahmebehälters vorzugsweise mit der zweiten Drehachse (28) zusammenfällt.

4. Verwendung nach Anspruch 1, wobei in der Mischvorrichtung die Halteeinrichtung (72, 88) einen insbesondere zylindrischen Aufnahmeraum (74, 90, 92) aufweist, in dem der Mischbehälter (40, 48, 76, 94) einsteckbar ist, und wobei vorzugsweise die Innenabmessungen des Aufnahmeraums (74, 90, 92) den Außenabmessungen des Mischbehälters (40, 48, 76, 94) entsprechen.

5. Verwendung nach Anspruch 4, wobei der Aufnahmeraum (74) mit einem Deckel (78) verschließbar ist, der eine Öffnung (80) zum Hindurchführen eines Mischbehälter-Halses (82) aufweist.

6. Verwendung nach Anspruch 4 oder 5, wobei die Mischvorrichtung ein insbesondere ringförmig ausgebildetes Dämpfungselement (86) aufweist, das an dem Mischbehälter (76) anliegt.

7. Verwendung nach einem oder mehreren der Ansprüche 1 und 4 bis 6, wobei in der Mischvorrichtung die Halteeinrichtung (88)
(i) als Aufnahmebehälter-Einsatz (72, 88) ausgebildet ist;
(ii) mehrere Aufnahmeräume (90) aufweist, wobei die Längsachse mindestens eines Aufnahmeraums (90) parallel zur zweiten Drehachse (28) und in einem Abstand zu dieser verläuft; und/oder
(iii) mehrere Aufnahmeräume (92) aufweist, wobei die Längsachse mindestens eines Aufnahmeraums (92) senkrecht zur zweiten Drehachse (28) verläuft.

8. Verwendung nach Anspruch 1, wobei in dem Mischbehälter die Öffnung (68) auf einer Längsachse (56) des ersten und/oder zweiten Aufnahmeraums (50, 52) angeordnet ist.

9. Verwendung nach Anspruch 1 oder 8, wobei in dem Mischbehälter
(i) der zweite Aufnahmeraum (52) innerhalb des ersten Aufnahmeraums (50) angeordnet ist;
(ii) der zweite Aufnahmeraum (52) kegelförmig ausgebildet ist, und wobei vorzugsweise die Öffnung (68) an der Kegelspitze (66) angeordnet ist; und/oder
(iii) der erste Aufnahmeraum (50) zylindrisch ist.

10. Verfahren zur Zertrümmerung von biologischen Proben, bevorzugt zur Gewebeaufarbeitung und zum Aufschluss von Zellen, umfassend die Behandlung der biologischen Proben in einer dualen asymmetrischen Zentriluge (DAZ).

11. Verfahren nach Anspruch 10, wobei bei dem Verfahren Zertrümmerungshilfen zugesetzt werden, bevorzugt Perlen aus Materialien wie Stahl, Achat, Korund und Glas, besonders bevorzugt Perlen mit einem Durchmesser von mindestens 3 mm.

12. Verfahren nach Anspruch 10 oder 11, das in Gefäßen mit einem Volumen von maximal 10 ml, bevorzugt von maximal 2 ml durchgeführt wird, insbesondere in Eppendorf®-Gefäßen oder Kryo-Vials.

13. Kit zur Durchführung des Verfahrens nach einem oder mehreren der Ansprüche 10 bis 12, umfassend eine Halteeinrichtung (72, 88) wie in Ansprüchen 1 und 4 bis 8 definiert.

## Claims

1. Use of a mixing device for chemical and/or biological substances comprising
a first driving device (12) for rotating a cantilever (16) around a first rotation axis (18),
a mixing vessel (40) for taking up the substances connected with the cantilever (16) and spaced apart from first rotation axis (18) and
a second driving device (30) for rotating the mixing vessel (40) around a second rotation axis (28) extending through the mixing vessel (40)
wherein the interior walls (46) of the mixing vessel (40) have different distances to the second rotation axis (28); and/or
wherein a holding device (72, 80) arranged within the uptake vessel (24) serves for holding the mixing vessel (40) in a positionally accurate manner in said uptake vessel (24); and/or
of a mixing vessel for a dual asymmetric centrifuge (DAC) comprising
a first uptake space (50) for taking up a first substance and at least one second uptake space (52) for taking up a second substance, wherein an opening (68) is provided in a separating wall (70) between the uptake spaces (50, 52) to enable the transfer of one of the substances into the other uptake space (50, 52) upon the occurrence of centrifugal forces; for manufacturing lipid-based nanoparticles, for intermixing single-phase or multi-phase biological substances, for the disruption of cells and for working up tissues in a dual asymmetric centrifuge (DAC).

2. The use according to claim 1, wherein the mixing vessel (40) in said mixing device has an essentially cylindrical design, and wherein preferably a longitudinal axis (47) of the mixing vessel (40) is arranged in an angle to the second rotation axis (28) and wherein the angle preferably is in the range of from 70 to 110°.

3. The use according to claim 1 or 2, wherein said mixing device has an uptake vessel (24) for taking up the mixing vessel (40), wherein the mixing vessel (40) is preferably cylindrical and the longitudinal axis of the uptake vessel preferably coincides with said second rotation axis (28).

4. The use according to claim 1, wherein the holding device (72, 88) in said mixing device has an uptake space (74, 90, 92), in particular a cylindrical one, into which the mixing vessel (40, 48, 76, 94) is insertable, and wherein the inner dimensions of the uptake space (74, 90, 92) are preferably the same as the outer dimensions of the mixing vessel (40, 48, 76, 94).

5. The use according to claim 4, wherein the uptake space (74) is sealable with a lid (78) having an opening (80) for passing through a mixing vessel neck (82).

6. The use according to claim 4 or 5, wherein said mixing device has a dampening element (86), in particular one having an annular design, which bears against the mixing vessel (76).

7. The use according to one or more of claims 1 and 4 to 6, wherein the holding device (88) in said mixing device
(i) is designed as an uptake vessel insert (72, 88);
(ii) has several uptake spaces (90) wherein the longitudinal axis of at least one uptake space (90) runs in parallel to the second rotation axis (28) and spaced apart therefrom; and/or
(iii) has several uptake spaces (92) wherein the longitudinal axis of at least one uptake space (92) runs perpendicular to the second rotation axis (28).

8. The use according to claim 1, wherein an opening (68) is provided in said mixing vessel on a longitudinal axis (56) of the first and/or second uptake space (50, 52).

9. The use according to claim 1 or 8, wherein in said mixing vessel:
(i) the second uptake space (52) is arranged within the first uptake space (50);
(ii) the second uptake space (52) has a conical design, and wherein the opening (68) is preferably arranged at the cone tip (66); and/or
(iii) the first uptake space (50) is cylindrical.

10. A process for crushing biological samples, preferably for working up tissue and for disrupting cells, comprising the treatment of said biological samples in a dual asymmetric centrifuge (DAC).

11. The process according to claim 10, wherein disintegrating aids, preferably beads made from materials such as steel, agate, corundum and glass, more preferably beads having a diameter of at least 3 mm, are added in said process.

12. The process according to claim 10 or 11, which is performed in vessels having a volume of maximally 10 ml, preferably maximally 2 ml, in particular in Eppendorf^{®} vessels or Cryovials.

13. A kit for performing the process according to one or more of claims 10 to 12, comprising a holding device (72, 88) as defined in claims 1 and 4 to 8.

## Revendications

1. Utilisation d'un dispositif de mélange pour substances chimiques et/ou biologiques, comprenant :
un premier dispositif d'entraînement (12) pour rotation d'une potence (16) autour d'un premier axe de rotation (18),
un récipient mélangeur (40) raccordé à la potence (16) à distance du premier axe de rotation (18) et destiné à recevoir les substances, et
un deuxième dispositif d'entraînement (30) pour rotation du récipient mélangeur (40) autour d'un deuxième axe de rotation (28) traversant le récipient mélangeur (40),
les parois internes (46) du récipient mélangeur (40) étant situées à des distances différentes par rapport au deuxième axe (28), et/ou un dispositif de maintien (72, 80), disposé à l'intérieur du réceptacle (24),
assurant le positionnement précis du récipient mélangeur (40) dans le réceptacle (24), et/ou
d'un récipient mélangeur pour une centrifugeuse asymétrique double (DAZ) comprenant un premier espace de réception (50) destiné à recevoir une première substance et au moins un deuxième espace de réception (52) destiné à recevoir une deuxième substance, une ouverture (68) étant prévue dans une paroi de séparation (70) entre les espaces de réception (50, 52) pour permettre, lors de l'apparition de forces centrifuges, un transfert de l'une des substances dans l'autre espace de réception (50, 52) pour la fabrication de nanoparticules à base de lipides,
pour le mélange de substances biologiques monophasées ou multiphasées pour le fractionnement cellulaire et le traitement de tissus dans une centrifugeuse asymétrique double (DAZ).

2. Utilisation selon la revendication 1, dans laquelle, dans le dispositif de mélange, le récipient mélangeur (40) a une configuration sensiblement cylindrique, et dans lequel de préférence un axe longitudinal (47) du récipient mélangeur (40) est disposé en formant un angle par rapport au deuxième axe de rotation (28), l'angle étant compris de préférence dans la plage entre 70 et 110°.

3. Utilisation selon la revendication 1 ou 2, dans laquelle le dispositif de mélange présente un réceptacle (24) destiné à recevoir le récipient mélangeur (40), le récipient mélangeur (40) étant de préférence cylindrique et l'axe longitudinal du réceptacle coïncidant de préférence avec le deuxième axe de rotation (28).

4. Utilisation selon la revendication 1, dans laquelle, dans le dispositif de mélange, le dispositif de maintien (72, 88) présente un espace de réception (74, 90, 92) en particulier cylindrique dans lequel peut être inséré le récipient mélangeur (40, 48, 76, 94), et dans laquelle, de préférence, les dimensions intérieures de l'espace de réception (74, 90, 92) correspondent aux dimensions extérieures du récipient mélangeur (40, 48, 76, 94).

5. Utilisation selon la revendication 4, dans laquelle l'espace de réception (74) peut être fermé au moyen d'un couvercle (78) qui présente une ouverture (80) permettant le passage d'un goulot (82) de récipient mélangeur.

6. Utilisation selon la revendication 4 ou 5, dans laquelle le dispositif de mélange présente un élément d'amortissement (86) en particulier de configuration annulaire qui repose contre le récipient mélangeur (76).

7. Utilisation selon une ou plusieurs des revendications 1 et 4 à 6, dans laquelle, dans le dispositif de mélange, le dispositif de maintien (88)
(i) est conçu sous la forme d'un insert de réceptacle (72, 88) ;
(ii) présente plusieurs espaces de réception (90), l'axe longitudinal d'au moins un espace de réception (90) s'étendant parallèlement au deuxième axe de rotation (28) et à distance de celui-ci ; et/ou
(iii) présente plusieurs espaces de réception (92), l'axe longitudinal d'au moins un espace de réception (92) s'étendant perpendiculairement au deuxième axe de rotation (28).

8. Utilisation selon la revendication 1, dans laquelle, dans le récipient mélangeur, l'ouverture (68) est disposée sur un axe longitudinal (56) du premier et/ou du deuxième espace de réception (50, 52).

9. Utilisation selon la revendication 1 ou 8, dans laquelle, dans le récipient mélangeur,
(i) le deuxième espace de réception (52) est disposé à l'intérieur du premier espace de réception (50) ;
(ii) le deuxième espace de réception (52) est de forme conique, l'ouverture (68) étant de préférence disposée au niveau de la pointe conique (66) ; et/ou
(iii) le premier espace de réception (50) est cylindrique.

10. Procédé de fragmentation d'échantillons biologiques, de préférence pour le traitement de tissus et pour la fragmentation de cellules, comprenant le traitement des échantillons biologiques dans une centrifugeuse asymétrique double (DAZ).

11. Procédé selon la revendication 10, dans lequel sont ajoutés, durant le procédé, des auxiliaires de fragmentation, de préférence des perles en matériaux tels que : acier, agate, corindon et verre, de façon particulièrement préférée des perles d'un diamètre d'au moins 3 mm.

12. Procédé selon la revendication 10 ou 11, qui est réalisé dans des récipients d'un volume maximum de 10 ml, de préférence d'un volume maximum de 2 ml, en particulier des tubes Eppendorf® ou Kryo.

13. Kit pour réaliser le procédé selon l'une ou plusieurs des revendications 10 à 12, comprenant un dispositif de maintien (72, 88) tel que défini dans les revendications 1 et 4 à 8.
